(19) Europäisches Patentamt
European Patent Office
Office européen des brevets

(11) EP 1 299 373 B1

(12) **EUROPÄISCHE PATENTSCHRIFT**

(45) Veröffentlichungstag und Bekanntmachung des Hinweises auf die Patenterteilung:
**12.01.2005 Patentblatt 2005/02**

(21) Anmeldenummer: **01947447.7**

(22) Anmeldetag: **06.07.2001**

(51) Int Cl.[7]: **C07D 311/58**, C07D 313/08, C07D 337/08, C07D 335/06, C07C 211/31, A61K 31/335, A61K 31/38, A61P 25/20

(86) Internationale Anmeldenummer:
**PCT/EP2001/007750**

(87) Internationale Veröffentlichungsnummer:
**WO 2002/008218 (31.01.2002 Gazette 2002/05)**

(54) **CYCLISCHE SUBSTITUIERTE AMINOMETHYLVERBINDUNGEN UND DIESE VERBINDUNGEN ENTHALTENDE ARZNEIMITTEL**

CYCLIC SUBSTITUTED AMINOMETHYL COMPOUNDS AND MEDICAMENTS CONTAINING SAID COMPOUNDS

COMPOSES AMINOMETHYLES CYCLIQUES SUBSTITUES ET MEDICAMENTS CONTENANT LESDITS COMPOSES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **10.07.2000 DE 10033459**

(43) Veröffentlichungstag der Anmeldung:
**09.04.2003 Patentblatt 2003/15**

(73) Patentinhaber: **Grünenthal GmbH**
**52078 Aachen (DE)**

(72) Erfinder:
- **ZIMMER, Oswald, Karl**
  **52152 Würselen (DE)**
- **KÖGEL, Babette-Yvonne**
  **52379 Langerwehe-Hamich (DE)**
- **STRASSBURGER, Wolfgang, Werner, Alfred**
  **52146 Würselen (DE)**

(56) Entgegenhaltungen:
**EP-A- 0 922 703**
**US-A- 3 956 391**
**US-A- 6 022 895**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 299 373 B1

## Beschreibung

Die vorliegende Anmeldung betrifft cyclische substituierte Aminomethylverbindungen, Verfahren zur ihrer Herstellung, Zwischenverbindungen dieser Verfahren, Arzneimittel, die mindestens eine der cyclischen substituierten Aminomethylverbindungen enthalten, die Verwendung der cyclischen substituierten Aminomethylverbindungen zur Herstellung eines Arzneimittels zur Behandlung von Schmerz, Harninkontinenz, Juckreiz, Tinnitus aurium und/oder Diarrhoe sowie diese Verbindungen enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Die biochemische und pharmakologische Charakterisierung von beispielsweise μ-, K- und δ-Subtypen dieser Rezeptoren hat die Hoffnung geweckt, daß subtypenspezifische Opioide über ein anderes Wirkungs-/Nebenwirkungsprofil als die klassischen Opioide wie z.B. Morphin verfügen. Morphin bindet selektiv an die sogenannten μ-Rezeptoren. Andererseits sind Verbindungen mit antinociceptivem Potential bekannt, die nicht oder kaum an μ-Rezeptoren binden und deren analgetische Wirkung ausschließlich oder überwiegend über δ-Rezeptoren vermittelt wird.

So offenbart die DE 197 55 480 A1 substituierte heterocyclische Benzocycloalkene, deren analgetische Wirksamkeit weitgehend oder ausschließlich über δ-Rezeptoren vermittelt wird. Substituierte Aminoverbindungen mit entsprechender biologischer Wirksamkeit sind aus der DE 198 05 370 A1 bekannt.

Der vorliegenden Erfindung liegt die Aufgabe zugrunde, analgetisch wirksame Verbindungen zur Verfügung zu stellen, die sich zur Schmerztherapie - insbesondere auch zur Therapie chronischer und neuropathischer Schmerzen - eignen. Darüber hinaus sollten diese Substanzen möglichst keine der Nebenwirkungen, die üblicherweise bei der Anwendung von Opioiden mit μ-Rezeptoraffinität auftreten, wie z.B. Übelkeit, Erbrechen, Abhängigkeit, Atemdepression oder Obstipation, hervorrufen.

Diese Aufgabe wird durch cyclische substituierte Aminomethylverbindungen der allgemeinen Formeln IA und/oder IB gelöst, die aufgabengemäß keine oder eine nur geringe Affinität zu μ-Rezeptoren zeigen und darüber hinaus überraschend in-vivo analgetisch wirken, obwohl sie in-vitro auch keine spezifische Wirksamkeit an δ-Opiatrezeptoren zeigen.

Bei den erfindungsgemäßen Verbindungen handelt es sich um cyclische substituierte Aminomethylverbindungen der allgemeinen Formeln IA und/oder IB

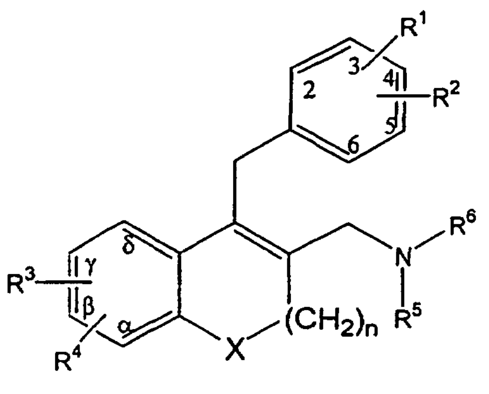

IA

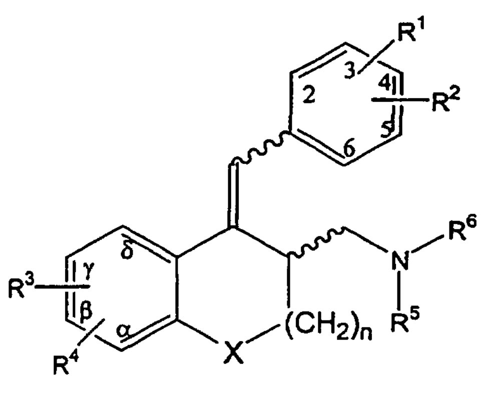

IB ,

worin

$R^1$ H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet,

| | |
|---|---|
| $R^2$ | H, F, Cl, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet, |
| $R^3$ und $R^4$ | unabhängig voneinander H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$, $CF_3$, O-Aryl, Aryl oder Heterocyclyl, jeweils in α-, β-, γ- und/oder δ-Stellung des aromatischen Ringes, bedeuten, |
| $R^5$ und $R^6$ | unabhängig voneinander $CH_3$, $C_{2-6}$-Alkyl, $C_{3-7}$-Cycloalkyl, $CH_2$-($C_{3-7}$-Cycloalkyl), Aryl, ($C_{1-6}$-Alkyl)-Aryl, Heterocyclyl oder ($C_{1-6}$-Alkyl)-Heterocyclyl bedeuten, |
| X | $CH_2$, O, S, SO oder $SO_2$ bedeutet, |
| n | 0, 1, 2 oder 3 ist, wenn X $CH_2$ bedeutet, und 1, 2 oder 3 ist, wenn X O, S, SO oder $SO_2$ bedeutet, |

und die Konfiguration der exocyclischen Doppelbindung in Verbindungen der allgemeinen Formel IB E- oder Z- ist, sowie ihre pharmazeutisch annehmbaren Salze.

Die erfindungsgemäßen Verbindungen zeigen ohne Affinität zu μ-Rezeptoren eine deutliche analgetische Wirkung, ohne zugleich eine spezifische Wirksamkeit an δ-Rezeptoren aufzuweisen. Es ist bislang nicht geklärt, in welcher Weise und über welche Opiatrezeptor-Subtypen die erfindungsgemäßen Verbindungen ihre analgetische Wirkung vermitteln.

Der Ausdruck "$C_{2-6}$-Alkyl" umfaßt im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 2, 3, 4, 5 oder 6 Kohlenstoffatomen, d.h. $C_{2-6}$-Alkanyle, $C_{2-6}$-Alkenyle und $C_{2-6}$-Alkinyle. Vorteilhaft ist $C_{2-6}$-Alkyl aus der Gruppe ausgewählt, die Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$), Butenyl, Butinyl, Pentenyl, Pentinyl, Hexenyl und Hexinyl umfaßt.

Der Ausdruck "$C_{3-7}$-Cycloalkyl" bedeutet für die Zwecke dieser Erfindung cyclische Kohlenwasserstoffe mit 3, 4, 5, 6 oder 7 Kohlenstoffatomen, die gesättigt oder ungesättigt, unsubstituiert oder ein- oder mehrfach substituiert sein können. Vorteilhaft ist $C_{3-7}$-Cycloalkyl aus der Gruppe ausgewählt, die Cyclopropyl, Cyclobutyl, Cyclopentyl, Cyclohexyl, Cycloheptyl, Cyclopentenyl, Cyclohexenyl und Cycloheptenyl enthält.

Der Ausdruck "Aryl" bedeutet im Sinne dieser Erfindung u.a. Phenyle, Naphthyle oder Anthracenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten in jeder beliebigen Position des Aryls sein können. Vorteilhafterweise ist Aryl aus der Gruppe ausgewählt, die Phenyl, p-Toluyl, p-Methoxyphenyl, Xylyl, 1-Naphthyl, 2-Naphthyl, 4-Biphenyl enthält. Bevorzugte Substituenten sind OH, F, Cl, Br, $C_{1-6}$-Alkyl, $C_{3-7}$-Cycloalkyl, $CH_2F$, $CHF_2$, $CF_3$, O-$C_{1-6}$-Alkyl, O-$C_{3-7}$-Cycloalkyl, O-$CH_2$-$C_{3-7}$-Cycloalkyl, Heterocyclyl, Phenyl, Naphthyl.

Der Ausdruck "Heterocyclyl" steht für einen 5-, 6- oder 7-gliedrigen cyclischen organischen Rest, der mindestens 1, ggf. auch 2, 3, 4 oder 5 Heteroatome, enthält, wobei die Heteroatome gleich oder verschieden sind und der cyclische Rest gesättigt, ungesättigt oder aromatisch, unsubstituiert oder ein- oder mehrfach substituiert sein kann. Der Heterocyclus kann auch Teil eines bi- oder polycyclischen Systems sein. Bevorzugte Heteroatome sind Stickstoff, Sauerstoff und Schwefel. Es ist bevorzugt, daß der Heterocyclyl-Rest ausgewählt ist aus der Gruppe, die Pyrrolyl, Furyl, Thienyl, Pyrazolyl, Imidazolyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl, Phenothiazinyl, Pyrrolidinyl, Piperidinyl, Piperazinyl, Morpholinyl enthält, wobei die Bindung an das Stickstoff-Atom, den/die Aryl-Ring/e, den Phenylring bzw. den aromatischen Ring der erfindungsgemäßen Verbindungen der allgemeinen Formeln IA und IB über jedes beliebige Ringglied des Heterocyclyl-Restes erfolgen kann.

Die Ausdrücke "$C_{1-6}$-Alkyl-Aryl" bzw. "$C_{1-6}$-Alkyl-Heterocyclyl" bedeuten für die Zwecke der vorliegenden Erfindung, daß $C_{1-6}$-Alkyl, Aryl und Heterocyclyl die oben definierten Bedeutungen haben und über eine $C_{1-6}$-Alkyl-Gruppe an den aromatischen Ring bzw. das Stickstoff-Atom des Aminomethylrestes der Verbindungen der Formel IA und/ oder IB gebunden sind.

Im Zusammenhang mit "Alkyl", "Alkanyl", "Alkenyl" und "Alkinyl" versteht man unter dem Begriff "substituiert" im Sinne dieser Erfindung die Substitution eines Wasserstoffrestes durch F, Cl, Br, I, $NH_2$, SH oder OH, wobei unter mehrfach substituierten Resten solche Reste zu verstehen sind, die sowohl an verschiedenen als auch an gleichen Atomen mehrfach, z.B. zwei- oder dreifach, substituiert sind, beispielsweise dreifach am gleichen C-Atom wie im Falle von $CF_3$ oder $-CH_2CF_3$ oder an verschiedenen Stellen wie im Falle von $-CH(OH)-CH=CH-CHCl_2$. Die Mehrfachsubstitution kann mit dem gleichen oder mit verschiedenen Substituenten erfolgen.

In Bezug auf "Aryl", "Alkyl-Aryl", "Heterocyclyl", "Alkyl-Heterocyclyl" sowie "Cycloalkyl" bzw. "$CH_2$-($C_{3-7}$-Cyclo-

alkyl)" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, $NH_2$, SH, OH, $CF_3$, $NO_2$, $SO_3H$, C(O)OH; =O oder =S; ein- oder mehrfach substituiertes oder unsubstituiertes $C_{1-6}$-Alkanyl, $C_{2-6}$-Alkenyl, $C_{2-6}$-Alkinyl, O-$C_{1-6}$-Alkyl, - C(O)O-$C_{1-6}$-Alkyl, -$C_{1-6}$-Alkyl-C(O)O-$C_{1-6}$-Alkyl; ein- oder mehrfach substituiertes oder unsubstituiertes Phenyl, Benzyl, Naphthyl oder Heterocyclyl; an einem oder ggf. verschiedenen Atomen. Die Mehrfachsubstitution erfolgt dabei mit dem gleichen oder mit unterschiedlichen Substituenten.

Die Reste $R^1$ und $R^2$ können jeweils in 2-, 3-, 4-, 5- oder 6-Position des Phenylringes vorgesehen sein, d.h. in ortho-, meta- oder para-Stellung.

Die Reste $R^3$ und $R^4$ können jeweils in $\alpha$-, $\beta$-, $\gamma$- oder $\delta$-Position des aromatischen Ringes vorgesehen sein.

X bedeutet in den erfindungsgemäßen Verbindungen der Formeln IA und IB $CH_2$ (Methylen), O (Sauerstoff), S (Schwefel), SO (Sulfoxid) oder $SO_2$ (Sulfon), während n für den Fall, daß X $CH_2$ bedeutet, 0, 1, 2 oder 3 ist, bzw. für den Fall, daß X O, S, SO oder $SO_2$ bedeutet, 1, 2 oder 3 ist. Wenn X = $CH_2$, sind die Verbindungen der allgemeinen Formeln IA und IB z.B. Indan- oder Inden-Derivate (n = 0), Dihydro- bzw. Tetrahydronaphthalin-Derivate (n = 1), Benzocycloheptyl-Derivate (n = 2) oder Benzocyclooctyl-Derivate (n = 3). Wenn X für Sauerstoff bzw. Schwefel steht, sind die erfindungsgemäßen Verbindungen z.B. für n = 2 Oxepin- bzw. Thiepin-Derivate. Wenn X = SO, liegen cyclische Sulfoxide, wenn X = $SO_2$, cyclische Sulfone vor.

Die erfindungsgemäßen cyclischen substituierten Aminomethylverbindungen der allgemeinen Formel IB können sowohl in der *E*- als auch in der Z-Konfiguration als auch als Gemisch beider Konfigurationen vorliegen. Dabei wird für die Zwecke dieser Erfindung unter E-Konfiguration jene stereochemische Anordnung verstanden, in der der mit $R^1$ und $R^2$ substituierte Phenylring und der mit $R^3$ und $R^4$ substituierte aromatische Ring *trans* zueinander sind, während in der *Z*-Konfiguration die beiden Ringe *cis* zueinander angeordnet sind:

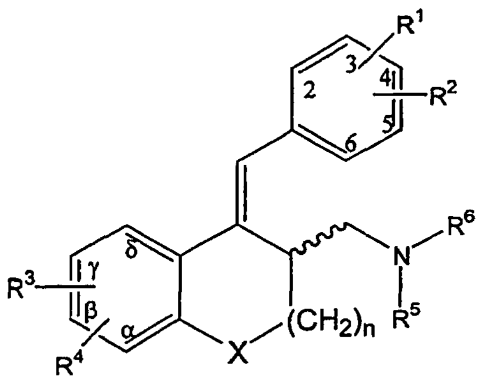

*E*-IB

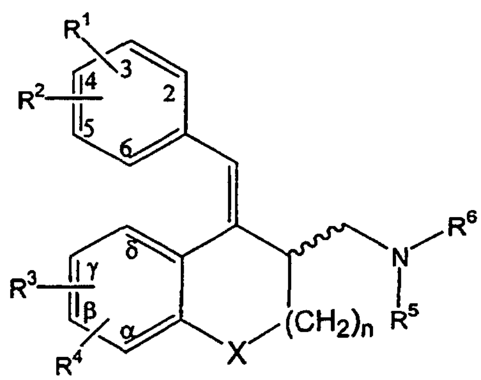

*Z*-IB .

Pharmazeutisch annehmbare Salze im Sinne dieser Erfindung sind solche Salze der erfindungsgemäßen Verbindungen gemäß der allgemeinen Formeln IA und/oder IB, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind die Hydrate der erfindungsgemäßen Verbindungen, die z.B. durch Kristallisation aus wäßriger Lösung erhalten werden können.

Eine Gruppe von bevorzugten Verbindungen der vorliegenden Erfindung wird von solchen cyclischen substituierten Aminomethylverbindungen der allgemeinen Formel IA gebildet, in denen unabhängig voneinander $R^1$ OH, O-$CH_3$ oder Cl, $R^2$ H oder Cl, $R^3$ H oder OH, $R^4$ H, $R^5$ und $R^6$ $CH_3$ und X $CH_2$, O, S oder SO bedeuten und n 1 oder 2 ist, sowie ihren pharmazeutisch annehmbaren Salze. Besonders bevorzugt sind dabei die Verbindungen der allgemeinen Formel IA, in denen unabhängig voneinander $R^1$ 3-OH, 2-O-$CH_3$, 3-O-$CH_3$ oder 4-Cl, $R^2$ H, 2-Cl oder 4-Cl, $R^3$ H, $\alpha$-OH oder $\beta$-OH, $R^4$ H, $R^5$ und $R^6$ $CH_3$ und X $CH_2$, O, S oder SO bedeuten und n 1 oder 2 ist, sowie deren pharmazeutisch annehmbaren Salze.

Eine weitere Gruppe von bevorzugten Verbindungen der vorliegenden Erfindung wird von solchen cyclischen substituierten Aminomethylverbindungen der allgemeinen Formel IB gebildet, in denen $R^1$ OH, O-$CH_3$ oder Cl, $R^2$, $R^3$ und $R^4$ H, $R^5$ und $R^6$ $CH_3$ und X $CH_2$, O oder S bedeuten, n 1 oder 2 ist und die Konfiguration der exocyclischen

Doppelbindung E- oder Z- ist, sowie ihren pharmazeutisch annehmbaren Salzen. Besonders bevorzugt sind dabei cyclische substituierte Aminomethylverbindungen der Formel IB, bei denen $R^1$ 3-OH, 2-O-$CH_3$, 3-O-$CH_3$ oder 4-Cl, $R^2$, $R^3$ und $R^4$ H, $R^5$ und $R^6$ $CH_3$ und X $CH_2$, O oder S sind, n 1 oder 2 ist und die Konfiguration der exocyclischen Doppelbindung *E*- oder *Z*- ist, sowie ihre pharmazeutisch annehmbaren Salze.

Darüber hinaus ist es bevorzugt, daß die erfindungsgemäßen Verbindungen der allgemeinen Formeln IA und/oder IB als Gemisch der Isomeren mit endocyclischer und exocyclischer Doppelbindung vorliegen, d.h. als Gemisch von Verbindungen, die sich nur durch die Position der aliphatischen Doppelbindung in dem die Gruppe X enthaltenden Ring bzw. außerhalb des die Gruppe X enthaltenden Ringes unterscheiden, bei denen aber die Definition von $R^1$ bis $R^6$, X und n übereinstimmt. Das Verhältnis der Isomeren in diesem Gemisch kann variieren. Z.B. kann das Verhältnis der endo- und der exo-Verbindungen der Formeln IA und IB in einem Bereich von 100:1 bis 1:100 liegen. Vorzugsweise beträgt das Verhältnis 1:1, 1:2, 2:1, 1:10, 10:1, 1:100 oder 100:1.

Die erfindungsgemäßen Verbindungen der allgemeinen Formeln IA und/oder IB können - soweit es sich bei ihnen um optisch aktive Substanzen handelt - in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Das gilt insbesondere für Verbindungen der allgemeinen Formel IB, die stets ein asymmetrisches Zentrum in der mit einem Stern * markierten allylischen Position zur exocyclischen Doppelbindung aufweisen:

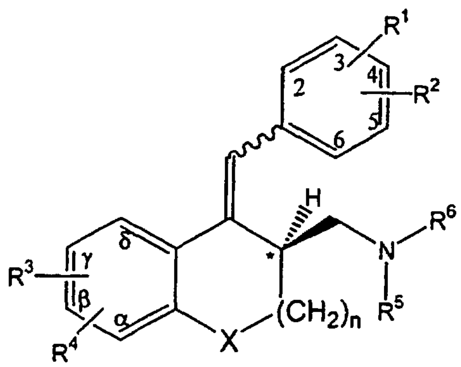

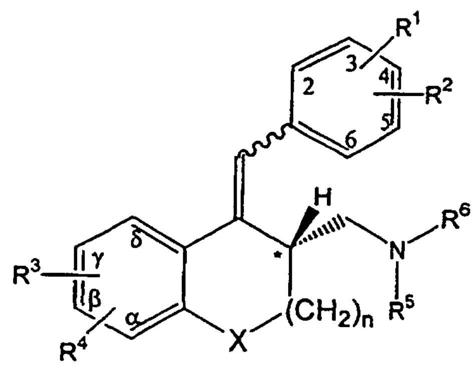

.

Die Mischungen können in jedem beliebigen Mischungsverhältnis der enthaltenen Enantiomeren bzw. Diastereomeren vorliegen. Vorzugsweise liegen die erfindungsgemäßen Verbindungen bzw. ihre pharmazeutisch annehmbaren Salze in enantiomerenreiner Form vor.

Besonders bevorzugt sind die folgenden erfindungsgemäßen Verbindungen:

[1-(4-Chlorbenzyl)-3,4-dihydro-naphth-2-ylmethyl]-dimethylamin,
3-(2-Dimethylaminomethyl-3,4-dihydro-naphth-1-yl-methyl)-phenol,
5-(4-Chlorbenzyl)-6-dimethylaminomethyl-7,8-dihydro-naphth-1-ol,
*E*-(5*RS*)-[5-(4-Chlorbenzyliden)-6,7,8,9-tetrahydro-5H-benzocyclohepten-6-ylmethyl]-dimethylamin,
*Z*-(4*RS*)[5-(4-Chlorbenzyliden)-2,3,4,5-tetrahydrobenzo[b]oxepin-4-ylmethyl]dimethylamin,
3-(4-Dimethylaminomethyl-2,3-dihydro-benzo[b]oxepin-5-ylmethyl)-phenol,
*E*-(4*RS*)-3-(4-Dimethylaminomethyl-3,4-dihydro-2H-benzo[b]oxepin-5-ylidenmethyl)-phenol oder
*Z*-(4*RS*)-3-(4-Dimethylaminomethyl-3,4-dihydro-2H-benzo[b]oxepin-5-ylidenmethyl)-phenol,

sowie ihre pharmazeutisch annehmbaren Salze, insbesondere ihre Hydrochloride, und zwar sowohl in racemischer als auch in nichtracemischer und in enantiomerenreiner Form. Insbesondere bevorzugt ist *E*-(4*RS*)-3-(4-Dimethylaminomethyl-3,4-dihydro-2H-benzo[b]oxepin-5-ylidenmethyl)-phenol in Form seines Hydrochlorids.

Die Erfindung betrifft auch ein Verfahren zur Herstellung der erfindungsgemäßen Verbindungen der allgemeinen Formeln IA und/oder IB

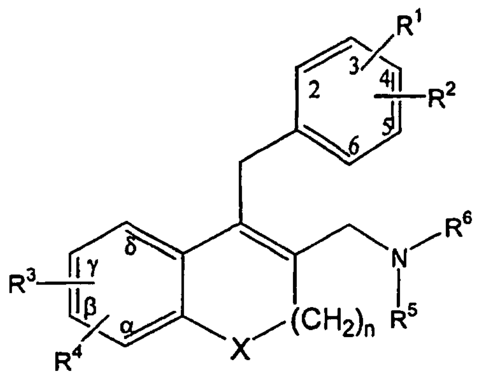
IA

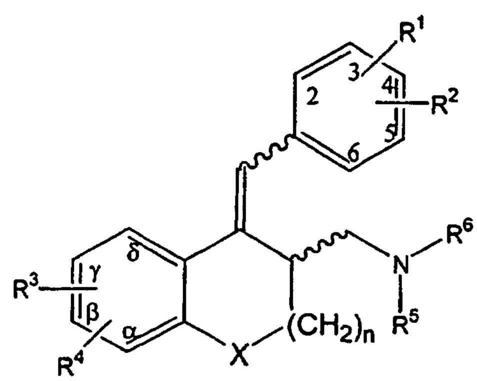
IB ,

worin

| | |
|---|---|
| $R^1$ | H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$ oder $CF_3$, jeweils in 2- ,3-, 4-, 5- oder 6-Stellung des Phenylringes, bedeutet, |
| $R^2$ | H, F, Cl, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet, |
| $R^3$ und $R^4$ | unabhängig voneinander H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$, $CF_3$, O-Aryl, Aryl oder Heterocyclyl, jeweils in α-, β-, γ- und/oder δ-Stellung des aromatischen Ringes, bedeuten, |
| $R^5$ und $R^6$ | unabhängig voneinander $CH_3$, $C_{2-6}$-Alkyl, $C_{3-7}$-Cycloalkyl, $CH_2$-($C_{3-7}$-Cycloalkyl), Aryl, ($C_{1-6}$-Alkyl)-Aryl, Heterocyclyl oder ($C_{1-6}$-Alkyl)-Heterocyclyl bedeuten, |
| X | $CH_2$, O, S, SO oder $SO_2$ bedeutet, |
| n | 0, 1, 2 oder 3 ist, wenn X $CH_2$ bedeutet, und 1, 2 oder 3 ist, wenn X O, S, SO oder $SO_2$ bedeutet, |

und die Konfiguration der exocyclischen Doppelbindung in Verbindungen der allgemeinen Formel IB *E*- oder *Z*- ist, wobei das erfindungsgemäße Verfahren durch einen Verfahrensschritt (a) gekennzeichnet ist, der die Umsetzung eines tertiären Alkohols der allgemeinen Formel II

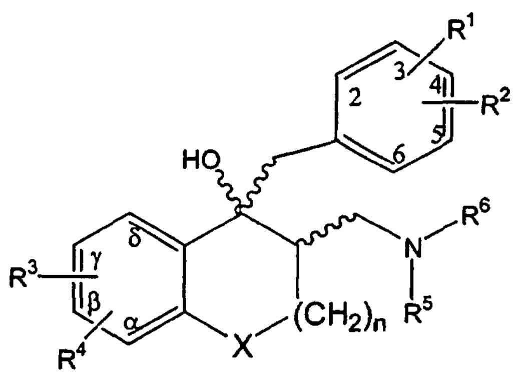
II ,

worin $R^1$ bis $R^6$, X und n wie oben definiert sind, mit einer Säure umfaßt.

Bevorzugt ist hierbei die Verwendung von halbkonzentrierten oder konzentrierten organischen oder anorganischen Säuren, insbesondere Salzsäure (HCl), z.B. 6N Salzsäure, wahlweise in einem wäßrigen oder einem organischen Lösungsmittel, wie z.B. Diethylether, konzentrierte Bromwasserstoffsäure (HBr), Bromwasserstoff in Eisessig (HBr/HOAc), z.B. eine 33 %ige Bromwasserstoff-Lösung in Eisessig, Methansulfonsäure, Methansulfonsäure mit Methionin sowie Ameisensäure.

Üblicherweise wird der Verfahrensschritt (a) bei einer Temperatur von etwa 0° C bis etwa 120° C ausgeführt.

Durch Auswahl einer geeigneten Säure kann auch die bevorzugte Bildung entweder des endo-Isomeren der Formel IA oder des/der exo-Isomeren der Formel IB erreicht werden. So bildet sich bei Verwendung von HBr in Eisessig

als Säure in dem Verfahrensschritt (a) bevorzugt die endo-Verbindung IA, während sich beispielsweise bei Einsatz von 6N HCl überwiegend die exo-Produkte IB bilden.

Selbstverständlich können die nach dem erfindungsgemäßen Verfahren gebildeten und nach Durchführung des Verfahrensschritts (a) in einem Gemisch vorliegenden Verbindungen der allgemeinen Formeln IA und IB auch mittels herkömmlicher Trennmethoden voneinander abgetrennt werden, wobei auch die Trennung des jeweiligen exo-*E*-Isomeren der Formel IB von dem entsprechenden exo-*Z*-Isomeren der Formel IB erreicht werden kann. Als geeignete Methoden seien beispielhaft chromatographische Trennverfahren, insbesondere Flüssigchromatographie-Verfahren unter Normaldruck oder erhöhtem Druck, bevorzugt MPLC- und HPLC-Verfahren, sowie Kristallisationsverfahren genannt. Darüber hinaus können auch die gebildeten Enantiomeren und/oder Diastereomeren der erfindungsgemäßen Verbindungen IA und IB mit Hilfe dieser und weiterer, im Stand der Technik bekannter Verfahren, z.B. HPLC an chiralen Phasen oder fraktionierte Kristallisation von mit optisch aktiven Säuren, wie (+)-Weinsäure, (-)-Weinsäure oder (+)-10-Camphersulfonsäure, gebildeten diastereomeren Salzen, voneinander getrennt werden.

Eine Bestimmung und Zuordnung der Stereochemie der erfindungsgemäß hergestellten Produkte, d.h. die Identifizierung der jeweiligen endo- und exo-Doppelbindungs- sowie der *E*- und *Z*-Isomeren, erfolgt mit Hilfe von im Stand der Technik bekannten Methoden, beispielsweise mittels im Stand der Technik wohlbekannter kernresonanzspektroskopischer (NMR-)Verfahren. So erfolgt z.B. die Unterscheidung der exo-Isomeren der Formel IB von den endo-Isomeren der Formel IA anhand der chemischen Verschiebung des bzw. der benzylischen Wasserstoffatome im $^1$H-NMR-Spektrum. Andererseits ist die Zuordnung der *E*-/*Z*-Isomerie über die aufgrund von Anisotropieeffekten differierende chemische Verschiebung der Phenyl-Ringprotonen im $^1$H-NMR-Spektrum möglich.

Eine bevorzugte Ausführungsform des erfindungsgemäßen Verfahrens ist dadurch gekennzeichnet, daß der Verfahrensschritt (a) die Umsetzung eines tertiären Alkohols der allgemeinen Formel II, in der mindestens einer der Reste $R^1$, $R^3$ und $R^4$ O-$CH_3$ bedeutet, zu Verbindungen der allgemeinen Formeln IA und/oder IB, in denen die Reste $R^1$, $R^3$ und $R^4$ OH bedeuten, wenn die entsprechenden Reste $R^1$, $R^3$ und $R^4$ in dem tertiären Alkohol der allgemeinen Formel II O-$CH_3$ bedeuten, mit einem Reagenz aus der Gruppe, die Bromwasserstoff in Eisessig, konzentrierte Bromwasserstoffsäure und Methansulfonsäure/Methionin enthält, umfaßt. Der Verfahrensschritt wird vorzugsweise bei einer Temperatur zwischen 0° C und 120° C, insbesondere bei einer Temperatur zwischen 20° C und 50° C ausgeführt.

Eine weitere bevorzugte Ausführungsform des erfindungsgemäßen Verfahrens ist dadurch gekennzeichnet, daß vor dem Verfahrensschritt (a) ein Verfahrensschritt (b) ausgeführt wird, der die Überführung eines tertiären Alkohols der allgemeinen Formel III

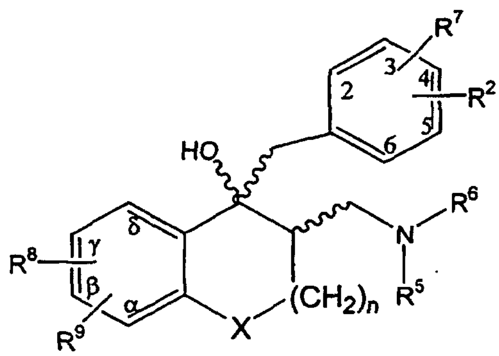

III ,

worin

| | |
|---|---|
| $R^2$, $R^5$, $R^6$, X und n | wie oben definiert sind, |
| $R^7$ | H, F, Cl, O-$CH_3$, O-($C_{2-6}$-Alkyl), O-($C_{3-7}$-Cycloalkyl), O-$CH_2$-Phenyl, O-Si$R^{10}R^{11}R^{12}$, wobei $R^{10}$, $R^{11}$ und $R^{12}$ unabhängig voneinander $CH_3$, $C_{2-6}$-Alkyl oder Phenyl sind, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet, |
| $R^8$ und $R^9$ | unabhängig voneinander H, F, Cl, O-$CH_3$, O-($C_{2-6}$-Alkyl), O- ($C_{3-7}$-Cycloalkyl), O-$CH_2$-Phenyl, O-Si$R^{10}R^{11}R^{12}$, wobei $R^{10}$, $R^{11}$ und $R^{12}$ unabhängig voneinander $CH_3$, $C_{2-6}$-Alkyl oder Phenyl sind, $CH_3$, $C_{2-6}$-Alkyl, $CH_2F$, $CHF_2$, $CF_3$, O-Aryl, Aryl oder Heterocyclyl, jeweils in α-, β-, γ- und/oder δ-Stellung des aromatischen Ringes, bedeuten, |

und mindestens einer der Reste $R^7$, $R^8$ und $R^9$

O-$CH_3$, O-($C_{2-6}$-Alkyl), O-($C_{3-7}$-Cycloalkyl), O-$CH_2$-Phenyl oder O-Si$R^{10}R^{11}R^{12}$ ist,

in einen tertiären Alkohol der allgemeinen Formel II, in der $R^1$, $R^3$ und $R^4$ jeweils OH ist, wenn der entsprechende Rest $R^7$, $R^8$ bzw. $R^9$ in der Formel III $O-CH_3$, $O-(C_{2-6}$-Alkyl), $O-(C_{3-7}$-Cycloalkyl), $O-CH_2$-Phenyl oder $O-SiR^{10}R^{11}R^{12}$ ist, umfaßt. Wenn mindestens einer der Reste $R^7$, $R^8$ und $R^9$ eine Silanyloxy-Gruppe (= $O-SiR^{10}R^{11}R^{12}$) darstellt, so steht $O-SiR^{10}R^{11}R^{12}$ bevorzugt für eine Trimethylsilyloxy- , tert.-Butyldiphenylsilyloxy- oder tert.-Butyldimethylsilyloxy-Gruppe.

Stehen $R^7$, $R^8$ und/oder $R^9$ für eine Benzyloxy-Gruppe (= $O-CH_2$-Phenyl), so umfaßt der Verfahrensschritt (b) zweckmäßig eine reduktive Debenzylierung mit katalytisch aktiviertem Wasserstoff. Als Katalysator können dabei u.a. Platin oder Palladium dienen, wobei das Übergangsmetall z.B. auf einem geeigneten Trägermaterial wie Aktivkohle absorbiert sein kann. Die Reaktion wird vorzugsweise in einem organischen Lösungsmittel, z.B. Essigsäure, Methanol, Ethanol, 2-Propanol, 1-Propanol, 1-Butanol, 2-Butanol oder tert.-Butanol, bei Drücken von 1 bar ($10^5$ Pa) bis 100 bar ($10^7$ Pa) und Temperaturen von etwa 20° C bis etwa 100° C durchgeführt. Vorzugsweise werden die tertiären Alkohole der allgemeinen Formel III dabei in Form eines ihrer Salze eingesetzt.

Stellen $R^7$, $R^8$ und/oder $R^9$ hingegen eine Silanyloxygruppe dar, so erfolgt der Verfahrensschritt (b) vorzugsweise entweder durch Behandlung des tertiären Alkohols III mit Fluorid-Anion, insbesondere Tetra-n-butylammoniumfluorid, in einem inerten Lösungsmittel, wie z.B. Tetrahydrofuran (THF), 1,4-Dioxan oder Diethylether, bei vorzugsweise Raumtemperatur, oder durch Einwirken von methanolischer Salzsäure.

Stehen $R^7$, $R^8$ und/oder $R^9$ in dem tertiären Alkohol der allgemeinen Formel III für Methoxy ($O-CH_3$), $O-(C_{2-6}$-Alkyl) oder $O-(C_{3-7}$-Cycloalkyl), so kann der Verfahrensschritt (b) durch Umsetzung der Verbindung III mit Diisobutylaluminiumhydrid in einem aromatischen Kohlenwasserstoff wie Toluol, vorzugsweise bei einer Temperatur zwischen etwa 60° C und 130° C, ausgeführt werden.

Die neuen und zur Herstellung der erfindungsgemäßen cyclischen substituierten Aminomethylverbindungen der allgemeinen Formeln IA und IB nützlichen tertiären Alkohole der allgemeinen Formeln II und III bilden einen Teil der vorliegenden Erfindung und können in einem weiteren Verfahrensschritt (c), der nachstehend erläutert wird, als Zwischenverbindungen erhalten und dann entweder mit bekannten Methoden gereinigt und isoliert oder direkt durch Ausführung der oben beschriebenen Verfahrensschritte (a) bzw. (a) und (b) in die Verbindungen der Formeln IA und/ oder IB überführt werden.

Dabei werden die tertiären Alkohole II bzw. III bevorzugt über einen Verfahrensschritt (c) erhalten, der die Umsetzung eines Ketons der allgemeinen Formel IV

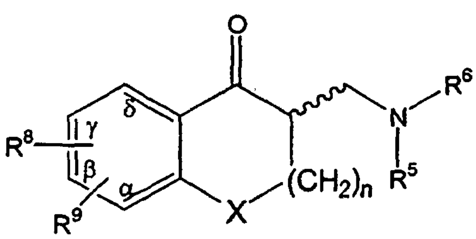

IV ,

worin $R^5$, $R^6$, $R^8$ und $R^9$, X und n wie oben definiert sind,
mit einer metallorganischen Verbindung der allgemeinen Formel V

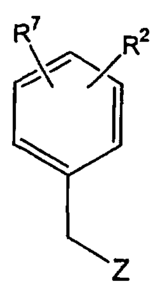

V ,

worin $R^2$ und $R^7$ wie oben definiert sind und Z MgCl, MgBr, MgI oder Li bedeutet, umfaßt.

Geeigenterweise wird der Verfahrensschritt (c) in einem Ether-Lösungsmittel, bevorzugt ein aliphatischer oder

cycloaliphatischer Ether, wie z.B. Diethylether oder THF, bei einer Temperatur von insbesondere zwischen -70° C und +60° C ausgeführt.

Die in dem Verfahrensschritt (c) eingesetzten Ketone der allgemeinen Formel IV sind als Mannich-Basen beispielsweise nach einem allgemeinen, z.B. aus der DE 197 55 480 A1 und der DE 198 05 370 A1 bekannten und u.a. von P. Horstmann und B. Unterhalt, *Arch. Pharm. Med. Chem.* **330,** 362-364 (1997), beschriebenen Verfahren aus dem entsprechenden Keton VI, Formaldehyd und dem Amin VII erhältlich (siehe Schema 1):

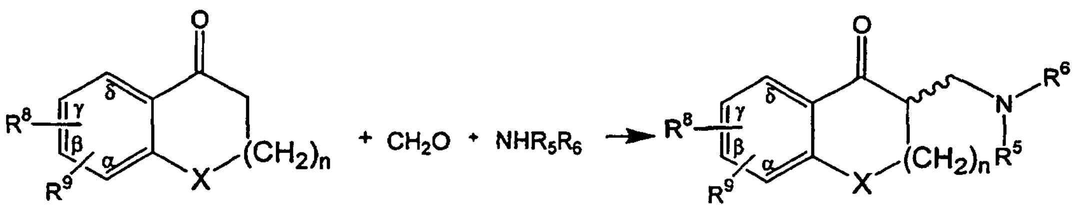


VI VII IV

Schema 1

In den Verbindungen der allgemeinen Formeln IV, VI und VII sind $R^5$, $R^6$, $R^8$, $R^9$, X und n wie oben definiert. Die Verbindungen der Formel VI sind, soweit sie nicht käuflich erhältlich sind, beispielsweise aus den korrespondierenden Carbonsäuren der allgemeinen Formel VIII durch intramolekulare Friedel-Crafts-Acylierung mittels einer Lewis-Säure oder einer Protonensäure, wie z.B. Polyphosphorsäure, zugänglich (siehe z.B. J. March: Advanced Organic Chemistry, 3. Ed., John Wiley & Sons, New York, Chichester, Brisbane, Toronto, Singapore (1985), Seiten 484 bis 487):

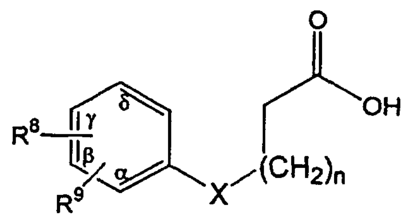

VIII

Die metallorganischen Verbindungen der allgemeinen Formel V mit Z = MgCl, MgBr, MgI oder Li sind, soweit sie nicht käuflich erhältlich sind, beispielsweise nach allgemein bekannten Verfahren durch Umsetzung der korrespondierenden Chloride, Bromide oder Iodide, d.h. Verbindungen der Formel V mit Z = Cl, Br oder I, mit Magnesium in einem inerten Lösungsmittel nach Grignard bzw. mit einem lithiumorganischen Reagenz, z.B. n-Butyllithium in n-Hexan, zugänglich. Die korrespondierenden Chloride, Bromide und Iodide der allgemeinen Formel V mit Z = Cl, Br und I sind ihrerseits entweder käuflich erhältlich oder z.B. durch Umsetzung der entsprechenden Benzylalkohole der Formel V mit Z = OH mit geeigneten Chlorierungs-, Bromierungs- bzw. Iodierungsreagenzien zugänglich (siehe beispielsweise J. March: Advanced Organic Chemistry, 3. Ed., John Wiley & Sons, New York, Chichester, Brisbane, Toronto, Singapore (1985), Seiten 382 bis 384).

Soweit in den Verbindungen der allgemeinen Formeln IV, V, VI und VIII $R^7$, $R^8$ und/oder $R^9$ O-$CH_3$, O-($C_{2-6}$-Alkyl), O-($C_{3-7}$-Cycloalkyl), O-$CH_2$-Phenyl oder O-Si$R^{10}R^{11}R^{12}$ bedeuten, können diese Alkoxy-, Benzyloxy- und Silanyloxy-Verbindungen durch Einführung geeigneter Schutzgruppen nach allgemein bekannten Verfahren, wie sie beispielsweise in T.W. Greene, P.G.M. Wuts: Protective Groups in Organic Synthesis, 1. Ed., John Wiley & Sons, New York, Chichester, Brisbane, Toronto, Singapore (1991), beschrieben sind, aus den entsprechenden Hydroxy-Verbindungen, d.h. solchen Verbindungen der allgemeinen Formeln IV, V, VI und VIII, in denen $R^7$, $R^8$ und/oder $R^9$ für OH stehen, erhalten werden.

Eine weitere bevorzugte Ausführungsform des erfindungsgemäßen Verfahrens umfaßt einen Verfahrensschritt (d), in dem Verbindungen der allgemeinen Formeln IA und/oder IB, in denen X gleich S bedeutet und $R^1$ bis $R^6$ und n wie oben definert sind, unter Verwendung eines Oxidationsmittels in die korrespondierenden Verbindungen IA und/oder IB mit X = SO und/oder $SO_2$ überführt werden. Die Oxidation des Sulfids (X = S) der allgemeinen Formel IA und/oder IB zum entsprechenden Sulfoxid (X = SO) kann u.a. mit einem Äquivalent Wasserstoffperoxid (30 gew.-%ige Lösung in Wasser) in einem geeigneten Lösungsmittel, z.B. Essigsäure, bei einer Temperatur zwischen etwa 20° C

und 60° C, die Oxidation zum Sulfon (X = $SO_2$) mit einem weiteren Äquivalent Wasserstoffperoxid ausgeführt werden. Weitere geeignete Oxidationsmittel sind u.a. Natriumperborat, t-Butylhypochlorit, Natriumperiodat und Kaliumhydrogenpersulfat (Oxone®) (siehe auch J. March: Advanced Organic Chemistry, 3. Ed., John Wiley & Sons, New York, Chichester, Brisbane, Toronto, Singapore (1985), Seiten 1089 und 1090).

Weiterhin ist es bevorzugt, daß zur Überführung von Verbindungen der allgemeinen Formeln IA und/oder IB, in denen mindestens einer der Reste $R^1$, $R^3$ und $R^4$ O-$CH_3$ bedeutet, in Verbindungen der allgemeinen Formeln IA und/oder IB, in denen der/die entsprechende/n Rest/e $R^1$, $R^3$ und $R^4$ OH bedeutet/bedeuten, ein Verfahrensschritt (e) nach dem Verfahrensschritt (a) und gegebenenfalls vor oder nach dem Verfahrensschritt (d) ausgeführt wird. Dieser Verfahrensschritt (e) kann z.B. durch Verwendung von Diisobutylaluminiumhydrid in einem aromatischen Kohlenwasserstoff, wie z.B. Toluol oder Xylol, bei einer Temperatur von 60° C bis 130° C ausgeführt werden. Eine alternative Durchführung des Schritts (e) besteht in der Umsetzung mit Methansulfonsäure/Methionin bei einer Temperatur von 20° C bis 50° C.

Bevorzugt ist es außerdem, daß nach dem Verfahrensschritt (a) bzw. (d) bzw. (e) ein Verfahrensschritt (f) ausgeführt wird, der die Überführung der erfindungsgemäßen Verbindungen IA und/oder IB in ihre pharmazeutisch annehmbaren Salze umfaßt. Der Verfahrensschritt (f) erfolgt dabei vorzugsweise durch Umsetzung der Verbindungen IA und/oder IB in flüssiger oder fester Phase mit anorganischen oder organischen Säuren - die gegebenenfalls auch an einer festen Phase gebunden sein können -, wie vorzugsweise Salzsäure, Bromwasserstoffsäure, Schwefelsäure, Phosphorsäure, Kohlensäure, p-Toluolsulfonsäure, Methansulfonsäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, die mit den erfindungsgemäßen Verbindungen IA und/oder IB physiologisch verträgliche und damit pharmazeutisch annehmbare Salze bilden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Vorzugsweise erfolgt die Salzbildung in einem organischen Lösungsmittel, wie z.B. Diethylether, Diisopropylether, Essigsäurealkylester, Aceton oder Methylethylketon. Die besonders bevorzugten Hydrochloride der erfindungsgemäßen Verbindungen sind insbesondere durch Ausführen des Verfahrensschritts (f) mit Trimethylchlorsilan in einem wasserhaltigen organischen Lösungsmittel zugänglich. Weiterhin sind die Hydrate, wie z.B. Mono-, Sesqui-, Di-, Triund Tetrahydrate, bevorzugte Salze der erfindungsgemäßen Verbindungen, die beispielsweise durch Kristallisation aus wäßriger Lösung erhalten werden können

In einem weiteren bevorzugten Verfahrensschritt (g), der nach dem Verfahrensschritt (a) oder vor oder nach dem Verfahrensschritt (d) oder vor oder nach dem Verfahrensschritt (e) oder nach dem Verfahrensschritt (f) erfolgen kann, werden die Verbindungen der allgemeinen Formeln IA und IB bzw. ihre pharmazeutisch annehmbaren Salze voneinander getrennt. Die Auftrennung kann mittels bekannter Trennverfahren erfolgen, wobei auch die Trennung des jeweiligen exo-*E*-Isomeren der Formel IB von dem entsprechenden exo-Z-Isomeren der Formel IB erreicht werden kann. Geeignete Methoden sind z.B. chromatographische Trennverfahren, insbesondere Flüssigchromatographie-Verfahren unter Normal- oder erhöhtem Druck, bevorzugt MPLC- und HPLC-Verfahren, sowie Verfahren der fraktionierten Kristallisation. Darüber hinaus können auch die gebildeten Enantiomeren und/oder Diastereomeren der erfindungsgemäßen Verbindungen IA und IB mit Hilfe dieser und weiterer, im Stand der Technik bekannter Verfahren, z.B. HPLC an chiralen Phasen oder fraktionierte Kristallisation von mit chiralen Säuren, wie (+)-Weinsäure, (-)-Weinsäure oder (+)-10-Camphersulfonsäure, gebildeten diastereomeren Salzen, voneinander getrennt werden.

Darüber hinaus ist ein Arzneimittel, das mindestens eine der erfindungsgemäßen cyclischen substituierten Aminomethylverbindungen der allgemeinen Formeln IA und/oder IB sowie ihrer pharmazeutisch annehmbaren Salze umfaßt, Gegenstand der Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid.

Ein weiterer Gegenstand der Erfindung ist die Verwendung mindestens einer erfindungsgemäßen cyclischen substituierten Aminomethylverbindung der allgemeinen Formeln IA und/oder IB, einschließlich ihrer Diastereomere oder Enantiomere, auch als Racemate oder Enantiomerengemisch in Form ihrer freien Base oder eines mit einer physiologisch verträglichen Säure gebildeten Salzes, insbesondere des Hydrochloridsalzes, zur Herstellung eines Arzneimittels zur Behandlung von Schmerz. Die erfindungsgemäßen Verbindungen haben sich in-vivo als analgetisch wirksam erwiesen. Zugleich binden die erfindungsgemäßen Verbindungen nicht oder kaum an μ-Rezeptoren und weisen keine spezifische Wirksamkeit an δ-Rezeptoren auf. So zeigt sich im μ-Opiatrezeptorbindungstest nach P. L. Wood (P. L. Wood et al., *Neuropharmacology*, **Vol. 20**, 1215 ff. (1981)), daß die erfindungsgemäßen Verbindungen der allgemeinen Formeln IA und IB nicht (0-20 % Hemmung bei einer Konzentration von 1 μM) oder nur sehr schwach ($K_i$ > 1 μM) an den μ-Rezeptor binden. Im δ-Opiatrezeptorbindungstest nach L. K. Vaughn (L. K. Vaughn et al., *Eur. J. Pharmacol.*, **Vol. 177,** 99 ff. (1990)) zeigen die erfindungsgemäßen Verbindungen keine spezifische Wirksamkeit am δ-Rezeptor (0-30 % Hemmung bei einer Konzentration von 1 μM; $K_i$ > 1 μM).

Überraschenderweise hat es sich herausgestellt, daß die erfindungsgemäßen cyclischen substituierten Aminomethylverbindungen der allgemeinen Formeln IA und/oder IB für weitere Indikationen, insbesondere zur Behandlung von Harninkontinenz, Juckreiz, Tinnitus aurium und/oder Diarrhoe sehr geeignet sind. Ein weiterer Gegenstand der Anmeldung ist daher die Verwendung mindestens einer erfindungsgemäßen cyclischen substituierten Aminomethylverbindung der allgemeinen Formeln IA und/oder IB einschließlich eines pharmazeutisch annehmbaren Salzes zur Herstellung eines Arzneimittels zur Behandlung von Harninkontinenz, Juckreiz, Tinnitus aurium und/oder Diarrhoe.

Darüber hinaus sind auch pharmazeutische Zusammensetzungen Gegenstand der vorliegenden Erfindung, die mindestens eine Verbindung der wie oben definierten allgemeinen Formeln IA und/oder IB oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen, Suspensionen, Sprays, Granulaten, Tabletten, Pellets, Patches, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen cyclischen substituierten Aminomethylverbindung der allgemeinen Formeln IA und/oder IB je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crosspovidon, Agar und Bentonit. Die Auswahl der Hilfsstoffe sowie die einzusetzenden Mengen derselben hängt davon ab, ob das Arzneimittel oral, peroral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße cyclische substituierte Aminomethylverbindungen der allgemeinen Formeln IA und/oder IB in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen cyclischen substituierten Aminomethylverbindungen der allgemeinen Formeln IA und/oder IB verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Formeln IA und/oder IB oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder Gummi, und pharmazeutischen Verdünnungsmitteln, wie z.B. Wasser, gemischt werden, um eine feste Präformulierungs-Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Präformulierungs-Zusammensetzung verteilt ist, so daß diese ohne weiteres in gleich wirksame Einheitsdosis-Formen, wie Tabletten, Pillen oder Kapseln, unterteilt werden kann. Die feste Präformulierungs-Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch beschichtet oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen cyclischen substituierten Aminomethylverbindung der allgemeinen Formeln IA und/oder IB appliziert.

Die nachfolgenden Beispiele dienen zur näheren Erläuterung der vorliegenden Erfindung.

Als stationäre Phase für die Säulenchromatographie wurde Kieselgel 60 (0,040 - 0,063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen bzw. Trennungen sind stets in Volumen/Volumen angegeben.

Die Synthese der Mannich-Basen der allgemeinen Formel IV erfolgte nach den von P. Horstmann und B. Unterhalt, *Arch. Pharm. Med. Chem*. **330,** 362-364 (1997), und in den Patentanmeldungen DE 198 05 370 A1 und DE 197 55 480 A1 beschriebenen Verfahren.

$^{1}$H-NMR-Untersuchungen zur Bestimmung der Stereochemie (exo/endo-Doppelbindung bzw. *E*/*Z*-Konfiguration) der erfindungsgemäßen Erfindungen wurden mit einem 300 MHz DPX Advance NMR-Gerät der Fa. Bruker durchgeführt.

Beispiel 1

[1-(3-Methoxybenzyl)-3,4-dihydro-naphth-2-ylmethyl]-dimethylamin-Hydrochlorid

1. Stufe:

(1RS,2RS)-2-Dimethylaminomethyl-1-(3-methoxybenzyl)-1,2,3,4-tetrahydro-naphth-1-ol

Zu einem frisch bereiteten Grignardreagenz aus 1,46 g Magnesiumspänen und 8,8 ml 3-Methoxybenzylchlorid in 50 ml trockenem Diethylether wurde bei 20°C unter Rühren eine Lösung von 8,13 g 2-Dimethylaminomethyl-3,4-dihydro-naphthalin-1-on in 20 ml trockenem Diethylether getropft. Man erhitzte das Reaktionsgemisch 3 Stunden zum Rückfluß, zersetzte durch Zutropfen von 30 ml einer gesättigten Ammoniumchloridlösung und extrahierte, nach Verdünnen mit destilliertem Wasser, dreimal mit je 50 ml Diethylether. Die Extrakte wurden mit einer gesättigten Natriumchloridlösung gewaschen, über Natriumsulfat getrocknet und i. Vak. eingedampft. Der ölige Rückstand wurde säulenchromatographisch mit Essigsäureethylester/Methanol = 20/1 als Elutionsmittel gereinigt. Dabei fielen 9,41 g (72,3 % d.Th.) der Titelverbindung als Diastereomerengemisch an.

2. Stufe:

[1-(3-Methoxybenzyl)-3,4-dihydro-naphth-2-ylmethyl]-dimethylamin-Hydrochlorid

6,51 g des Produkts aus Stufe 1 wurden mit 75 ml einer Lösung von Bromwasserstoff in Eisessig (33 % HBr) eine Stunde bei 60°C gerührt. Dann wurde i. Vak. eingedampft und der Rückstand in 150 ml Wasser aufgenommen. Man alkalisierte mit Kaliumcarbonat und extrahierte dreimal mit je 50 ml Dichlormethan. Die Extrakte wurden mit einer gesättigten Natriumchloridlösung gewaschen, über Natriumsulfat getrocknet und i.Vak. eingedampft. Die so erhaltene Rohbase der Titelverbindung wurde mit Trimethylchlorsilan/Wasser in 2-Butanon in das Hydrochlorid überführt.

| | |
|---|---|
| Ausbeute: | 5,81 g (84,5 % d.Th.) |
| Schmelzpunkt: | 202 -204°C |

Beispiel 2

[1-(4-Chlorbenzyl)-3,4-dihydro-naphth-2-ylmethyl]-dimethylamin-Hydrochlorid

Die Titelverbindung wurde unter Anwendung der in Beispiel 1 beschriebenen Verfahrensweise unter Einsatz von 4-Chlorbenzylchlorid in Stufe 1 anstelle der 3-Methoxyverbindung erhalten.

Schmelzpunkt: 247 - 248°C

Beispiel 3

3-(2-Dimethylaminomethyl-3,4-dihydro-naphth-1-ylmethyl)-phenol-Hydrochlorid

A: 4,71 g des Produkts aus Beispiel 1, Stufe 1, in 60 ml einer Lösung von Bromwasserstoff in Eisessig wurden 5 Stunden zum Rückfluß erhitzt. Dann wurde im Vakuum eingedampft, der Rückstand in 50 ml Wasser aufgenommen, durch Zugabe von 1 N Natronlauge neutralisiert, zuletzt mit Ammoniumhydroxid alkalisiert. Man extrahierte dreimal mit je 30 ml Essigsäureethylester, trocknete über Natriumsulfat und dampfte im Vakuum ein. Die zurückbleibende Rohbase der Titelverbindung wurde mit Trimethylchlorsilan/Wasser in 2-Butanon in das Hydrochlorid überführt.

Ausbeute: 3,75 g (78,4 % d.Th.)
Schmelzpunkt: 151 - 153°C (Zers.)

Die Titelverbindung ließ sich auch auf folgendem Weg erhalten:

B: Eine Suspension von 2,06 g des Produkts aus Beispiel 1 in 20 ml trockenem Toluol wurde mit 40 ml einer Lösung von Diisobutylaluminiumhydrid in Toluol (20 Gew.-%) versetzt und das Gemisch 12 Stunden zum Rückfluß erhitzt. Nach dem Abkühlen wurde durch Zutropfen von Ethanol, zuletzt vermischt mit Wasser, zersetzt und im Vakuum eingedampft. Den Rückstand versetzte man mit 30 ml Methanol und filtrierte über Filtererde. Das Filtrat wurde eingedampft und die so erhaltene Rohbase der Titelverbindung mit Trimethylchlorsilan/Wasser in 2-Butanon in das Hydrochlorid überführt, dessen Schmelzverhalten mit dem des oben erhaltenen Produkts identisch war.

Ausbeute: 1,58 g (79,6 % d.Th.)

Beispiel 4

Unter Anwendung der in Beispiel 3, Methode A, beschriebenen Verfahrensweise und unter Einsatz entsprechender tertiärer Alkohole erhielt man analog:

4a: 5-(4-Chlorbenzyl)-6-dimethylaminomethyl-7,8-dihydro-naphth-2-ol-Hydrochlorid
Schmelzpunkt: 110°C (Zers.)

4b: [1-(2,4-Dichlorbenzyl)-3,4-dihydro-naphth-2-ylmethyl]-dimethylamin-Hydrochlorid
Schmelzpunkt: 208 - 209°C

4c: 5-(4-Chlorbenzyl)-6-dimethylaminomethyl-7,8-dihydro-naphth-1-ol-Hydrochlorid
Schmelzpunkt: 136 - 139°C

4d: [5-(4-Chlorbenzyl)-8,9-dihydro-7H-benzocyclohepten-6-ylmethyl]-dimethylamin-Hydrochlorid
Schmelzpunkt: 240 - 243°C

4e: *E*-(5RS)-[5-(4-Chlorbenzyliden)-6,7,8,9-tetrahydro-5H-benzocyclohepten-6-ylmethyl]-dimethylamin-Hydrochlorid
Schmelzpunkt: 181 - 183°C
Als Produkte desselben Ansatzes wurden die Beispielsverbindungen 4d und 4e in Form der freien Basen säulenchromatographisch mit Essigsäureethylester/Methanol = 9/1 als Elutionsmittel getrennt.

4f: 6-Dimethylaminomethyl-5-(3-hydroxybenzyl)-7,8-dihydro-naphth-2-ol-Hydrochlorid
Schmelzpunkt: 89°C (Zers.)

Beispiel 5

[1-(2-Methoxybenzyl)-3,4-dihydro-naphth-2-ylmethyl]-dimethylamin-Hydrochlorid (5a) und *E*-(2RS)-[1-(2-Methoxybenzyliden)-1,2,3,4-tetrahydro-naphth-2-ylmethyl]-dimethylamin-Hydrochlorid (5b)

Eine Lösung von 7,06 g (1RS, 2RS)-2-Dimethylaminomethyl-1-(2-methoxybenzyl)-1,2,3,4-tetrahydronaphth-1-ol (hergestellt analog Beispiel 1, Stufe 1, aus 2-Methoxybenzylchlorid und 2-Dimethylaminomethyl-3,4-dihydronaphthalin-1-on) in 45 ml Ameisensäure wurde 3 Stunden bei 20°C gerührt. Man verdünnte mit 100 ml Wasser und alkalisierte durch portionsweise Zugabe von Kaliumcarbonat bis pH 9. Es wurde dreimal mit je 50 ml Dichlormethan extrahiert. Die Extrakte wurden mit gesättigter Natriumchloridlösung gewaschen, über Natriumsulfat getrocknet und im Vakuum eingedampft. Der Rückstand wurde säulenchromatographisch mit Essigsäureethylester als Elutionsmittel aufgetrennt, wobei die beiden Titelverbindungen als Basen anfielen, die mit Trimethylchlorsilan/Wasser in 2-Butanon in die Hydrochloride überführt wurden.

| | | |
|---|---|---|
| 5a: | Ausbeute: | 3,12 g (51,5 % d.Th.) |
| | Schmelzpunkt: | 181 - 183°C |
| 5b: | Ausbeute: | 2,38 g (39,3 % d.Th.) |
| | Schmelzpunkt: | 228 - 230°C |

Beispiel 6

3-(4-Dimethylaminomethyl-2,3-dihydro-benzo[b]oxepin-5-ylmethyl)-phenol-Hydrochlorid (6a),

*E*-(4RS)3-(4-Dimethylaminomethyl-3,4-dihydro-2H-benzo[b]oxepin-5-ylidenmethyl)-phenol-Hydrochlorid (6b) und *Z*-(4RS)3-(4-Dimethylaminomethyl-3,4-dihydro-2H-benzo[b]oxepin-5-ylidenmethyl)-phenol-Hydrochlorid (6c)

Eine Mischung aus 4,78 g (4RS, 5RS)-4-Dimethylaminomethyl-5-(3-methoxybenzyl)-2,3,4,5-tetrahydrobenzo [b]oxepin-5-ol (hergestellt aus 3-Methoxybenzylmagnesiumchlorid und 4-Dimethylaminomethyl-3,4-dihydro-2H-benzo [b]oxepin-5-on analog Beispiel 1, Stufe 1), 28 ml Methansulfonsäure und 3,20 g Methionin wurde 4 Tage bei 40°C gerührt. Dann wurde mit Eis versetzt und vorsichtig mit Natriumhydrogencarbonat alkalisiert. Man extrahierte dreimal mit je 30 ml Essigsäureethylester, wusch mit einer gesättigten Natriumchloridlösung, trocknete über Natriumsulfat und dampfte im Vakuum ein. Das so erhaltene rohe Produktgemisch wurde säulenchromatographisch mit Essigsäureethylester aufgetrennt und die einzelnen Verbindungen mit Trimethylchlorsilan/Wasser in 2-Butanon in ihre Hydrochloride überführt.

| | | |
|---|---|---|
| 6a: | Ausbeute: | 0,88 g (18,1 % d. Th.) |
| | Schmelzpunkt: | 194 - 196°C |
| 6b: | Ausbeute: | 0,76 g (15,7 % d.Th.) |
| | Schmelzpunkt: | 120°C (Zers.) |
| 6c: | Ausbeute: | 1,29 g (26,5 % d.Th.) |
| | Schmelzpunkt: | 154°C (Zers.) |

Beispiel 7

[5-(4-Chlorbenzyl)-2,3-dihydro-benzo[b]oxepin-4-ylmethyl]-dimethylamin-Hydrochlorid (7a),

*E*-(4RS)[5-(4-Chlorbenzyliden)-2,3,4,5-tetrahydro-benzo[b]oxepin-4-ylmethyl]-dimethylamin-Hydrochlorid (7b) und *Z*-(4RS)[5-(4-Chlorbenzyliden-2,3,4,5-tetrahydro-benzo[b]oxepin-4-ylmethyl]-dimethylamin-Hydrochlorid (7c)

7,27 g (4RS, 5RS)-5-(4-Chlorbenzyl)-4-dimethylaminomethyl-2,3,4,5-tetrahydrobenzo[b]oxepin-5-ol wurden analog der in Beispiel 6 beschriebenen Verfahrensweise mit 43 ml Methansulfonsäure und 4,81 g Methionin zur Reaktion gebracht. Nach ebenfalls analoger Aufarbeitung, Produkttrennung und Salzbildung wurden die Titelverbindungen in Form weißer Kristalle erhalten.

| | | |
|---|---|---|
| 7a: | Ausbeute: | 3,07 g (40,1 % d.Th.) |

| | | |
|---|---|---|
| | Schmelzpunkt: | 215 - 217°C |
| 7b: | Ausbeute: | 0,93 g (12,1 % d.Th.) |
| | Schmelzpunkt: | 92°C (Zers.) |
| 7c: | Ausbeute: | 1,69 g (22,1 % d.Th.) |
| | Schmelzpunkt: | 162- 164°C |

Beispiel 8

2-Chlor-5-(4-dimethylaminomethyl-2,3-dihydro-benzo[b]oxepin-5-ylmethyl)-phenol-Hydrochlorid

Aus 1,85 g (4RS, 5RS)-5-(4-Chlor-3-methoxybenzyl)-4-dimethylaminomethyl-2,3,4,5-tetrahydro-benzo[b]oxepin-5-ol, 10 ml Methansulfonsäure und 1,12 g Methionin wurden unter Anwendung der in Beispiel 6 beschriebenen Verfahrensweise 0,80 g (42,7 % d.Th.) der Titelverbindung erhalten.

Schmelzpunkt: 220 - 222°C

Beispiel 9

3-(4-Dimethylaminomethyl-2,3-dihydro-benzo[b]thiepin-5-ylmethyl)-phenol-Hydrochlorid (9a) und *Z*-(4RS)-3-(4-Dimethylaminomethyl-3,4-dihydro-2H-benzo[b]thiepin-5-ylidenmethyl)-phenol-Hydrochlorid (9b)

Aus 8,00 g (4RS, 5RS)-4-Dimethylaminomethyl-5-(3-methoxybenzyl)-2,3,4,5-tetrahydro-benzo[b]thiepin-5-ol, 45 ml Methansulfonsäure und 5,11 g Methionin wurden unter Anwendung der in Beispiel 6 beschriebenen Verfahrensweise, nach säulenchromatographischer Trennung der Produkte mit Essigsäureethylester/Methanol = 3/1 als Elutionsmittel und Überführen in die Hydrochloride die Titelverbindungen erhalten.

| | | |
|---|---|---|
| 9a: | Ausbeute: | 3,43 g (42,2 % d.Th.) |
| | Schmelzpunkt: | 92 % (Zers.) |
| 9b: | Ausbeute: | 1,57 g (19,4 % d.Th.) |
| | Schmelzpunkt: | 209 - 210°C |

Beispiel 10

(1RS)-3-(4-Dimethylaminomethyl-1-oxo-2,3-dihydro-benzo[b]thiepin-5-ylmethyl)-phenol-Hydrochlorid

0,60 g des Produkts aus Beispiel 9a in 6 ml Essigsäure und 0,5 ml einer wäßrigen Lösung von Wasserstoffperoxid (30 Gew.-% $H_2O_2$) wurden 2 Stunden bei 20°C gerührt. Man verdünnte mit 30 ml Wasser und alkalisierte zunächst mit 3N Natronlauge, dann mit Kaliumcarbonat bis zu einem pH-Wert von 8. Es wurde dreimal mit je 20 ml Essigsäureethylester extrahiert. Die vereinigten Extrakte wurden mit gesättigter Natriumchloridlösung gewaschen, über Natriumsulfat getrocknet und im Vakuum eingedampft. Der Rückstand wurde mit Trimethylchlorsilan/Wasser in 2-Butanon in das Hydrochlorid überführt. Man erhielt so 0,53 g (84,5 % d.Th.) der Titelverbindung.

Schmelzpunkt: 96°C (Zers.)

Beispiel 11

[5-(4-Chlorbenzyl)-2,3-dihydro-benzo[b]thiepin-4-ylmethyl]-dimethylamin-Hydrochlorid

3,00 g (4RS, 5RS)-5-(4-Chlor-benzyl)-4-dimethylaminomethyl-2,3,4,5-tetrahydro-benzo[b]thiepin-5-ol (hergestellt, wie in Beispiel 1, Stufe 1, beschrieben, aus (4RS)-4-Dimethylaminomethyl-3,4-dihydro-2H-benzo[b]thiepin-5-on und 4-Chlorbenzylmagnesiumchlorid), 17 ml Methansulfonsäure und 1,90 g Methionin wurden 7 Tage bei 50°C gerührt. Nach Aufarbeitung, Reinigung und Überführen in das Hydrochlorid, wie in Beispiel 6 beschrieben, erhielt man 1,87 g (59,4 % d.Th.) der Titelverbindung.

Schmelzpunkt: 223 - 224°C

Beispiel 12

(1R,S)-[5-(4-Chlorbenzyl)-1-oxo-2,3-dihydro-benzo[b]thiepin-4-ylmethyl]-dimethylamin-Hydrochlorid

0,25 g des Produkts aus Beispiel 11 wurden unter Anwendung der in Beispiel 10 beschriebenen Verfahrensweise in 2,3 ml Essigsäure mit 0,2 ml einer wäßrigen Lösung von Wasserstoffperoxid (30 Gew.-% $H_2O_2$) oxidiert. Nach ebenfalls analoger Aufarbeitung und Hydrochloridbildung erhielt man 0,21 g (80,6 % d.Th.) der Titelverbindung.

Schmelzpunkt: 227 - 229°C

Beispiel 13

*E*-(4RS)-[5-(3-Methoxybenzyliden)-2,3,4,5-tetrahydro-benzo[b]oxepin-4-ylmethyl]-dimethylamin-Hydrochlorid

1,00 g (4RS, 5RS)-4-Dimethylaminomethyl-5-(3-methoxybenzyl)-2,3,4,5-tetrahydro-benzo[b]oxepin-5-ol (siehe Beispiel 6) und 48 ml 6N Salzsäure wurden 24 h bei 20°C und 8 h bei 50°C gerührt. Dann wurde mit 6N Natronlauge alkalisiert und dreimal mit je 20 ml Essigsäureethylester extrahiert. Man wusch die Extrakte mit einer gesättigten Natriumchloridlösung, trocknete über Natriumsulfat und dampfte im Vakuum ein. Der Rückstand wurde säulenchromatographisch mit Essigsäureethylester als Elutionsmittel gereinigt. Nach Überführen ins Hydrochlorid mit einer Lösung von Chlorwasserstoff in Diethylether erhielt man 0,72 g (68,6 % d.Th.) der Titelverbindung.

Schmelzpunkt: 170 - 172°C

Beispiel 14

*E*-(4RS)-[5-(3-Methoxybenzyliden)-2,3,4,5-tetrahydro-benzo[b]thiepin-4-ylmethyl]-dimethylamin-Hydrochlorid (14a) und sein Z-Isomer 14b

5,00 g (4RS, 5RS)-4-Dimethylaminomethyl-5-(3-methoxybenzyl)-2,3,4,5-tetrahydro-benzo[b]thiepin-5-ol (siehe Beispiel 9) wurden nach der in Beispiel 13 beschriebenen Verfahrensweise mit 230 ml 6N Salzsäure umgesetzt. Nach ebenfalls analoger Aufarbeitung, säulenchromatographischer Trennung der Doppelbindungsisomeren mit Essigsäureethylester/Methanol = 3/1 und Überführen in das Hydrochlorid wurden die Titelverbindungen in Form weißer Kristalle erhalten.

| | | |
|---|---|---|
| 14a: | Ausbeute: | 1,10 g (20,9 % d.Th.) |
| | Schmelzpunkt: | 166 - 169°C |
| 14b: | Ausbeute: | 2,56 g (48,7 % d.Th.) |
| | Schmelzpunkt: | 164 - 167°C |

Pharmazeutische Formulierung eines erfindungsgemäßen Arzneimittels

1 g des Hydrochlorids der Verbindung 6b (*E*-(4RS)3-(4-Dimethylaminomethyl-3,4-dihydro-2H-benzo[b]oxepin-5-ylidenmethyl)-phenol-Hydrochlorid) wurde in 1 l Wasser für Injektionszwecke bei Raumtemperatur gelöst und anschließend durch Zugabe von NaCl (Natriumchlorid) auf isotone Bedingungen eingestellt.

Pharmakologische Untersuchung der erfindungsgemäßen Verbindungen

Die antinociceptive Wirksamkeit der erfindungsgemäßen Verbindungen wurde im mit Phenylchinon induzierten Writhing-Test an der Maus, modifiziert nach I.C. Hendershot, J. Forsaith, J. Pharmacol. Exp. Ther. **125,** 237-240 (1959), untersucht. Dazu wurden männliche NMRI-Mäuse mit einem Gewicht von 25-30 g eingesetzt. Gruppen von 10 Tieren pro Substanzdosis wurden 10 Minuten nach intravenöser Gabe einer erfindungsgemäßen Verbindung 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzählers wurde die Anzahl der schmerzinduzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt.

Aus der dosisabhängigen Abnahme der Writhingreaktionen im Vergleich zu parallel untersuchten Tiergruppen, denen keine erfindungsgemäße Verbindung appliziert wurde, wurden mittels Regressionsanalyse (Auswerteprogramm Martens EDV Service, Eckental) die $ED_{50}$-Werte der Writhingreaktion berechnet.

Die überwiegende Zahl der Substanzen wurde in der Standarddosis von 10 mg/kg getestet. Die prozentuale Hemmung (% Hemmung) der Writhingreaktionen durch eine Substanz wurde dann nach folgender Formel berechnet:

$$\% \text{ Hemmung} = 100 - \left( \frac{\text{Writhingreaktion behan. Tiere}}{\text{Writhingreaktion Kontrolle}} \text{ X } 100 \right)$$

("behan." = behandelte).

Die Ergebnisse sind für ausgewählte Verbindungen in Tabelle 1 zusammengefaßt.

Tabelle 1

| Beispiel Nr. | Writhing-Test, Maus, i.v. Abnahme der Writhingreaktionen oder $ED_{50}$ [mg/kg] |
|---|---|
| 2 | 6,85 (61,5 %) |
| 3 | 93 % |
| 4e | 69 % |
| 4c | 84 % |
| 7c | 79 % |
| 6a | 95 % |
| 6b | 91 % [2,15 mg/kg] |
| 6c | 78 % |

Die in Tabelle 1 wiedergegebenen Ergebnisse zeigen, daß die erfindungsgemäßen Verbindungen, insbesondere Beispielsverbindung Nr. 6b (*E*-(4RS)3-(4-Dimethylaminomethyl-3,4-dihydro-2H-benzo[b]oxepin-5-ylidenmethyl)-phenol-Hydrochlorid), die bereits bei einer Dosis von 2,15 mg/kg Körpergewicht 91 % Hemmung hervorruft, in-vivo analgetisch wirksam sind. Zugleich binden die erfindungsgemäßen Verbindungen nicht oder kaum an μ-Rezeptoren und weisen keine spezifische Wirksamkeit an δ-Rezeptoren auf.

## Patentansprüche

1. Cyclische substituierte Aminomethylverbindungen der allgemeinen Formel IA und/oder IB

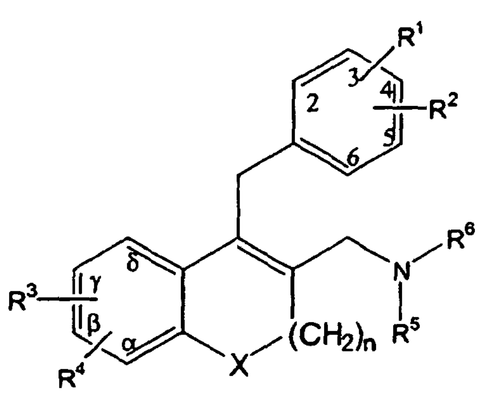

IA

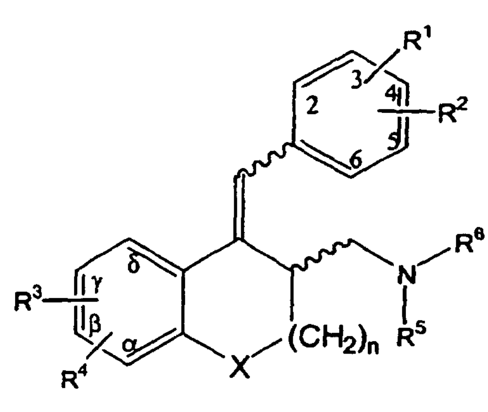

IB ,

worin

$R^1$ H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet,

$R^2$ H, F, Cl, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet,

$R^3$ und $R^4$ unabhängig voneinander H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$, $CF_3$, O-Aryl, Aryl oder Heterocyclyl, jeweils in $\alpha$-, $\beta$-, $\gamma$- und/oder $\delta$-Stellung des aromatischen Ringes, bedeuten,

$R^5$ und $R^6$ unabhängig voneinander $CH_3$, $C_{2-6}$-Alkyl, $C_{3-7}$-Cycloalkyl, $CH_2$-($C_{3-7}$-Cycloalkyl), Aryl, ($C_{1-6}$-Alkyl) -Aryl, Heterocyclyl oder ($C_{1-6}$-Alkyl)-Heterocyclyl bedeuten,

X $CH_2$, O, S, SO oder $SO_2$ bedeutet,

n 0, 1, 2 oder 3 ist, wenn X $CH_2$ bedeutet, und 1, 2 oder 3 ist, wenn X O, S, SO oder $SO_2$ bedeutet,

und die Konfiguration der exocyclischen Doppelbindung in Verbindungen der allgemeinen Formel IB *E*- oder *Z*- ist, sowie ihre pharmazeutisch annehmbaren Salze.

**2.** Cyclische substituierte Aminomethylverbindungen der allgemeinen Formel IA nach Anspruch 1, worin unabhängig voneinander $R^1$ OH, O-$CH_3$ oder Cl, $R^2$ H oder Cl, $R^3$ H oder OH, $R^4$ H, $R^5$ und $R^6$ $CH_3$ und X $CH_2$, O, S oder SO bedeuten und n 1 oder 2 ist, sowie ihre pharmazeutisch annehmbaren Salze.

**3.** Cyclische substituierte Aminomethylverbindungen der allgemeinen Formel IA nach einem der Ansprüche 1 oder 2, worin unabhängig voneinander $R^1$ 3-OH, 2-O-$CH_3$, 3-O-$CH_3$ oder 4-Cl, $R^2$ H, 2-Cl oder 4-Cl, $R^3$ H, $\alpha$-OH oder $\beta$-OH, $R^4$ H, $R^5$ und $R^6$ $CH_3$ und X $CH_2$, O, S oder SO bedeuten und n 1 oder 2 ist, sowie ihre pharmazeutisch annehmbaren Salze.

**4.** Cyclische substituierte Aminomethylverbindungen der allgemeinen Formel IB nach Anspruch 1, worin $R^1$ OH, O-$CH_3$ oder Cl, $R^2$, $R^3$ und $R^4$ H, $R^5$ und $R^6$ $CH_3$ und X $CH_2$, O oder S bedeuten, n 1 oder 2 ist und die Konfiguration der exocyclischen Doppelbindung E- oder Z-ist, sowie ihre pharmazeutisch annehmbaren Salze.

**5.** Cyclische substituierte Aminomethylverbindungen der allgemeinen Formel IB nach einem der Ansprüche 1 oder 4, worin $R^1$ 3-OH, 2-O-$CH_3$, 3-O-$CH_3$ oder 4-Cl, $R^2$, $R^3$ und $R^4$ H, $R^5$ und $R^6$ $CH_3$ und X $CH_2$, O oder S bedeuten, n 1 oder 2 ist und die Konfiguration der exocyclischen Doppelbindung *E*- oder *Z*- ist, sowie ihre pharmazeutisch annehmbaren Salze.

**6.** Cyclische substituierte Aminomethylverbindungen nach einem der Ansprüche 1 bis 5, wobei die Verbindungen als Gemisch der Isomeren mit endocyclischer Doppelbindung nach der allgemeinen Formel IA und mit exocyclischer Doppelbindung nach der allgemeinen Formel IB vorliegen.

**7.** Cyclische substituierte Aminomethylverbindungen der allgemeinen Formeln IA und/oder IB nach einem der Ansprüche 1 bis 6 in Form ihrer Racemate, in Form der reinen Enantiomeren oder in Form von Mischungen der Enantiomeren in einem beliebigen Mischungsverhältnis, sowie ihre pharmazeutisch annehmbaren Salze.

**8.** Cyclische substituierte Aminomethylverbindungen gemäß einem der Ansprüche 1 bis 7, wobei die Aminoverbindungen ausgewählt sind aus
[1-(4-Chlorbenzyl)-3,4-dihydro-naphth-2-ylmethyl]-dimethylamin,
3-(2-Dimethylaminomethyl-3,4-dihydro-naphth-1-yl-methyl)-phenol,
5-(4-Chlorbenzyl)-6-dimethylaminomethyl-7,8-dihydro-naphth-1-ol,
*E*-(5*RS*)-[5-(4-Chlorbenzyliden)-6,7,8,9-tetrahydro-5H-benzocyclohepten-6-ylmethyl]-dimethylamin,
*Z*-(4*RS*)-[5-(4-Chlorbenzyliden)-2,3,4,5-tetrahydrobenzo[b]oxepin-4-ylmethyl]-dimethylamin,
3-(4-Dimethylaminomethyl-2,3-dihydro-benzo[b]oxepin-5-ylmethyl)-phenol,
*E*-(4*RS*)-3-(4-Dimethylaminomethyl-3,4-dihydro-2H-benzo[b]oxepin-5-ylidenmethyl)-phenol oder
*Z*-(4*RS*)-3-(4-Dimethylaminomethyl-3,4-dihydro-2H-benzo[b]oxepin-5-ylidenmethyl)-phenol,

sowie ihre pharmazeutisch annehmbaren Salze, insbesondere ihre Hydrochloride.

**9.** Verfahren zur Herstellung von Verbindungen der allgemeinen Formeln IA und/oder IB

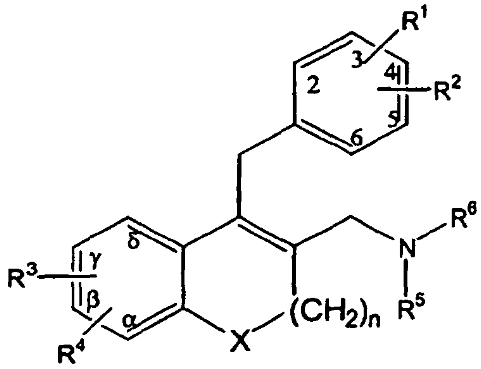

IA

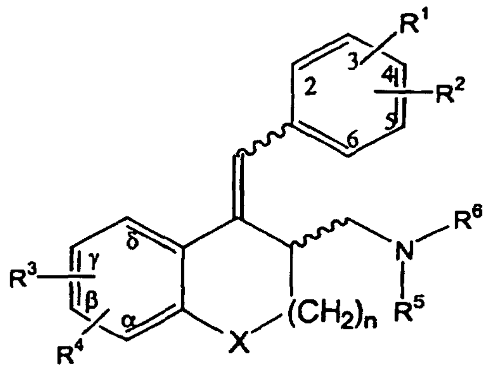

IB ,

worin

- $R^1$ H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet,
- $R^2$ H, F, Cl, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet,
- $R^3$ und $R^4$ unabhängig voneinander H, F, Cl, OH, O-$CH_3$, O-($C_{2-6}$-Alkyl), O-($C_{3-7}$-Cycloalkyl), $CH_3$, $C_{2-6}$-Alkyl, $CH_2F$, $CHF_2$, $CF_3$, O-Aryl, Aryl oder Heterocyclyl, jeweils in α-, β-, γ- und/oder δ-Stellung des aromatischen Ringes, bedeuten,
- $R^5$ und $R^6$ unabhängig voneinander $CH_3$, $C_{2-6}$-Alkyl, $C_{3-7}$-Cycloalkyl, $CH_2$-($C_{3-7}$-Cycloalkyl), Aryl, ($C_{1-6}$-Alkyl)-Aryl, Heterocyclyl oder ($C_{1-6}$-Alkyl)-Heterocyclyl, bedeuten,
- X $CH_2$, O, S, SO oder $SO_2$ bedeutet,
- n 0, 1, 2 oder 3 ist, wenn X $CH_2$ bedeutet, und 1, 2 oder 3 ist, wenn X O, S, SO oder $SO_2$ bedeutet,

und die Konfiguration der exocyclischen Doppelbindung in Verbindungen der allgemeinen Formel IB *E*- oder *Z*- ist, **gekennzeichnet durch** einen Verfahrensschritt (a), der die Umsetzung eines tertiären Alkohols der allgemeinen Formel II

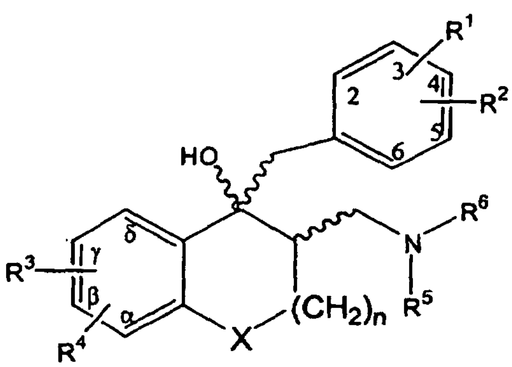

II ,

worin $R^1$ bis $R^6$, X und n wie oben definiert sind, mit einer Säure umfaßt.

**10.** Verfahren nach Anspruch 9, **dadurch gekennzeichnet, daß** der Verfahrensschritt (a) die Umsetzung eines tertiären Alkohols der allgemeinen Formel II, in der mindestens einer der Reste $R^1$, $R^3$ und $R^4$ O-$CH_3$ bedeutet, zu Verbindungen der allgemeinen Formeln IA und/oder IB, in denen die Reste $R^1$, $R^3$ und $R^4$ OH bedeuten, wenn die entsprechenden Reste $R^1$, $R^3$ und $R^4$ in dem tertiären Alkohol der allgemeinen Formel II O-$CH_3$ bedeuten, mit einem Reagenz aus der Gruppe, die Bromwasserstoff in Eisessig, konzentrierte Bromwasserstoffsäure und Methansulfonsäure/Methionin enthält, umfaßt.

**11.** Verfahren nach Anspruch 9, **dadurch gekennzeichnet, daß** vor dem Verfahrensschritt (a) ein Verfahrensschritt (b) ausgeführt wird, der die Überführung eines tertiären Alkohols der allgemeinen Formel III

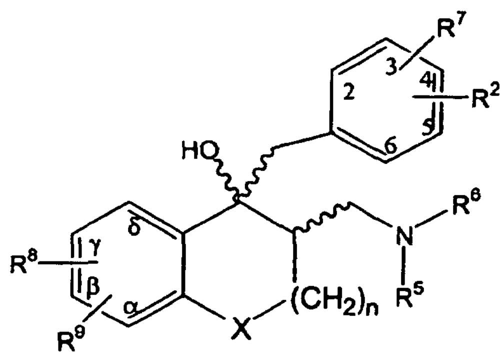

III ,

worin

| | |
|---|---|
| $R^2$, $R^5$, $R^6$, X und n | wie in Anspruch 9 definiert sind, |
| $R^7$ | H, F, Cl, O-$CH_3$, O-($C_{2-6}$-Alkyl), O-($C_{3-7}$-Cycloalkyl), O-$CH_2$-Phenyl, O-Si$R^{10}R^{11}R^{12}$, wobei $R^{10}$, $R^{11}$ und $R^{12}$ unabhängig voneinander $CH_3$, $C_{2-6}$-Alkyl oder Phenyl sind, $CH_3$, $C_{2-6}$-Alkyl, $CH_2F$, $CHF_2$ oder $CF_3$, jeweils in 2-, 3-, 4-, 5- oder 6-Stellung des Phenylringes, bedeutet, |
| $R^8$ und $R^9$ | unabhängig voneinander H, F, Cl, O-$CH_3$, O-($C_{2-6}$-Alkyl), O-($C_{3-7}$-Cycloalkyl), O-$CH_2$-Phenyl, O- Si$R^{10}R^{11}R^{12}$, wobei $R^{10}$, $R^{11}$ und $R^{12}$ unabhängig voneinander $CH_3$, $C_{2-6}$-Alkyl oder Phenyl sind, $CH_3$, $C_{2-6}$-Alkyl, $CH_2F$, $CHF_2$, $CF_3$, O-Aryl, Aryl oder Heterocyclyl, jeweils in $\alpha$-, $\beta$-, $\gamma$- und/oder $\delta$-Stellung des aromatischen Ringes, bedeuten, |

und mindestens einer der Reste $R^7$, $R^8$ und $R^9$

O-$CH_3$, O-($C_{2-6}$-Alkyl), O-($C_{3-7}$-Cycloalkyl), O-$CH_2$-. Phenyl oder O-Si$R^{10}R^{11}R^{12}$ ist,

in einen tertiären Alkohol der allgemeinen Formel II nach Anspruch 9, in der $R^1$, $R^3$ und $R^4$ jeweils OH ist, wenn der entsprechende Rest $R^7$, $R^8$ bzw. $R^9$ in Formel III O-$CH_3$, O-($C_{2-6}$-Alkyl), O-($C_{3-7}$-Cycloalkyl), O-$CH_2$-Phenyl oder O-Si$R^{10}R^{11}R^{12}$ ist,
umfaßt.

**12.** Verfahren nach einem der Ansprüche 9 bis 11, **dadurch gekennzeichnet, daß** zur Herstellung der tertiären Alkohole der allgemeinen Formeln II bzw. III vor den Verfahrensschritten (a) bzw. (b) ein Verfahrensschritt (c) ausgeführt wird, der die Umsetzung eines Ketons der allgemeinen Formel IV

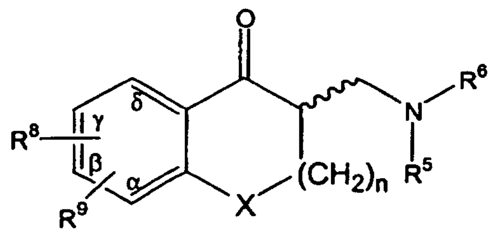

IV ,

worin $R^5$, $R^6$, X und n wie in Anspruch 9 und $R^8$ und $R^9$ wie in Anspruch 11 definiert sind, mit einer metallorganischen Verbindung der allgemeinen Formel V

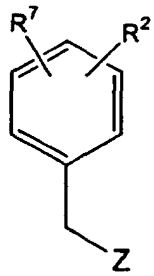

V ,

worin $R^2$ wie in Anspruch 9 und $R^7$ wie in Anspruch 11 definiert sind und Z MgCl, MgBr, MgI oder Li bedeutet, umfaßt.

**13.** Verfahren nach einem der Ansprüche 9 bis 12, **dadurch gekennzeichnet, daß** Verbindungen der allgemeinen Formeln IA und/oder IB mit X = S in einem Verfahrensschritt (d) in Verbindungen der allgemeinen Formeln IA und/oder IB mit X = SO und/oder $SO_2$ unter Verwendung eines Oxidationsmittels überführt werden.

**14.** Verfahren nach einem der Ansprüche 9 bis 13, **dadurch gekennzeichnet, daß** nach dem Verfahrensschritt (a) und gegebenenfalls vor oder nach dem Verfahrensschritt (d) ein Verfahrensschritt (e) ausgeführt wird, der die Überführung von Verbindungen der allgemeinen Formel IA und/oder IB, in denen mindestens einer der Reste $R^1$, $R^3$ und $R^4$ O-$CH_3$ bedeutet, in Verbindungen der allgemeinen Formeln IA und/oder IB, in denen der/die entsprechende/n Rest/e $R^1$, $R^3$ und $R^4$ OH bedeutet/bedeuten, umfaßt.

**15.** Verfahren nach einem der Ansprüche 9 bis 14, **dadurch gekennzeichnet, daß** nach dem Verfahrensschritt (a) bzw. (d) bzw. (e) ein Verfahrensschritt (f) ausgeführt wird, der die Überführung der Verbindungen der allgemeinen Formeln IA und/oder IB in ihre pharmazeutisch annehmbaren Salze umfaßt.

**16.** Verfahren nach einem der Ansprüche 9 bis 15, **dadurch gekennzeichnet, daß** nach dem Verfahrensschritt (a) bzw. vor oder nach dem Verfahrensschritt (d) bzw. vor oder nach dem Verfahrensschritt (e) bzw. nach dem Verfahrensschritt (f) ein Verfahrensschritt (g) ausgeführt wird, der die Trennung der Verbindungen der allgemeinen Formeln IA und IB sowie ggf. ihrer pharmazeutisch annehmbaren Salze umfaßt.

**17.** Tertiärer Alkohol der allgemeinen Formel II oder III

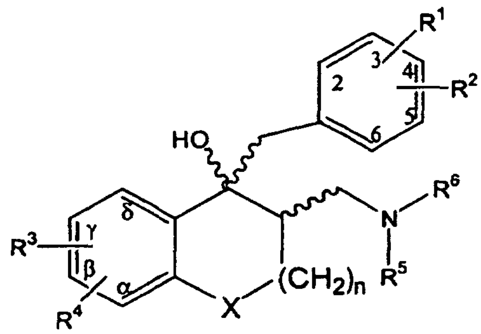

II

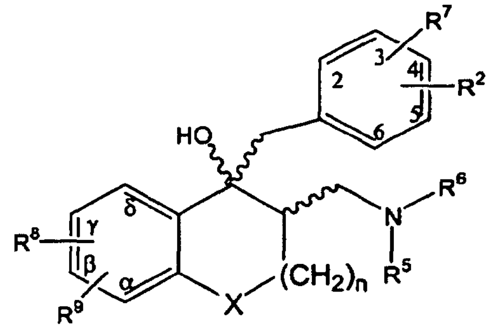

III ,

worin $R^1$ bis $R^6$, X und n wie in Anspruch 9 und $R^7$ bis $R^9$ wie in Anspruch 11 definiert sind.

**18.** Arzneimittel, enthaltend mindestens eine Verbindung der allgemeinen Formeln IA und/oder IB oder eines ihrer pharmazeutisch annehmbaren Salze nach einem der Ansprüche 1 bis 8.

**19.** Verwendung einer Verbindung der allgemeinen Formeln IA und/oder IB oder eines ihrer pharmazeutisch annehmbaren Salze nach einem der Ansprüche 1 bis 8 zur Herstellung eines Arzneimittels zur Behandlung von Schmerz.

**20.** Verwendung einer Verbindung der allgemeinen Formeln IA und/oder IB oder eines ihrer pharmazeutisch annehmbaren Salze nach einem der Ansprüche 1 bis 8 zur Herstellung eines Arzneimittels zur Behandlung von Harninkontinenz, Juckreiz, Tinnitus aurium und/oder Diarrhoe.

**21.** Pharmazeutische Zusammensetzung, die mindestens eine Verbindung der allgemeinen Formeln IA und/oder IB oder eines ihrer pharmazeutisch annehmbaren Salze nach einem der Ansprüche 1 bis 8 und einen oder mehrere pharmazeutische Hilfsstoffe enthält.

## Claims

**1.** Cyclic substituted aminomethyl compounds of the general formula IA and/or IB

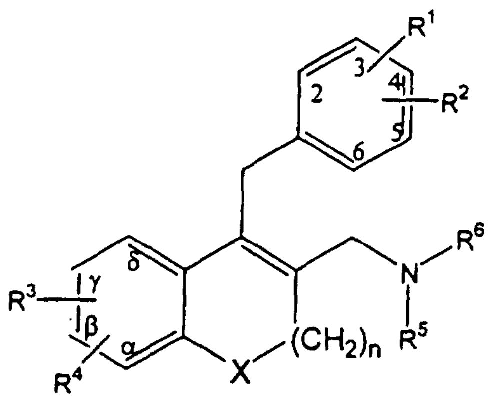

IA

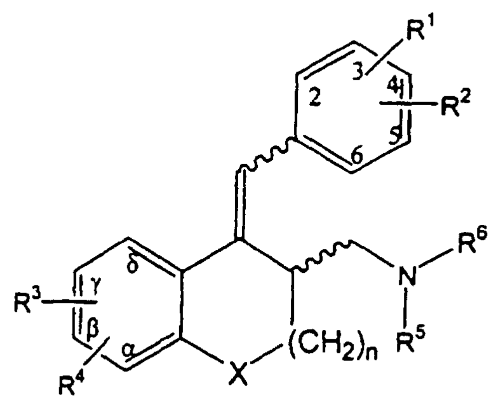

IB ,

wherein

$R^1$ denotes H, F, Cl, OH, O-$CH_3$, O-($C_{2-6}$-alkyl), O-($C_{3-7}$-cycloalkyl), $CH_3$, $C_{2-6}$-alkyl, $CH_2F$, $CHF_2$ or $CF_3$, in each case in the 2-, 3-, 4-, 5- or 6-position of the phenyl ring,

$R^2$ denotes H, F, Cl, $CH_3$, $C_{2-6}$-alkyl, $CH_2F$, $CHF_2$ or $CF_3$, in each case in the 2-, 3-, 4-, 5- or 6-position of the phenyl ring,

$R^3$ and $R^4$ independently of one another denote H, F, Cl, OH, O-$CH_3$, O-($C_{2-6}$-alkyl), O-($C_{3-7}$-cycloalkyl), $CH_3$, $C_{2-6}$-alkyl, $CH_2F$, $CHF_2$, $CF_3$, O-aryl, aryl or heterocyclyl, in each case in the α-, β-, γ- and/or δ-position of the aromatic ring,

$R^5$ and $R^6$ independently of one another denote $CH_3$, $C_{2-6}$-alkyl, $C_{3-7}$-cycloalkyl, $CH_2$-($C_{3-7}$-cycloalkyl), aryl, ($C_{1-6}$-alkyl) -aryl, heterocyclyl or ($C_{1-6}$-alkyl)-heterocyclyl,

X denotes $CH_2$, O, S, SO or $SO_2$,

n is 0, 1, 2 or 3 if X denotes $CH_2$ and is 1, 2 or 3 if X denotes O, S, SO or $SO_2$,

and the configuration of the exocyclic double bond in compounds of the general formula IB is E or Z, and their pharmaceutically acceptable salts.

**2.** Cyclic substituted aminomethyl compounds of the general formula IA according to claim 1, wherein, independently of one another, $R^1$ denotes OH, O-$CH_3$ or Cl, $R^2$ denotes H or Cl, $R^3$ denotes H or OH, $R^4$ denotes H, $R^5$ and $R^6$ denote $CH_3$ and X denotes $CH_2$, O, S or SO and n is 1 or 2, and their pharmaceutically acceptable salts.

**3.** Cyclic substituted aminomethyl compounds of the general formula IA according to one of claims 1 or 2, wherein, independently of one another, $R^1$ denotes 3-OH, 2-O-$CH_3$, 3-O-$CH_3$ or 4-Cl, $R^2$ denotes H, 2-Cl or 4-Cl, $R^3$ denotes H, α-OH or β-OH, $R^4$ denotes H, $R^5$ and $R^6$ denote $CH_3$ and X denotes $CH_2$, O, S or SO and n is 1 or 2, and their pharmaceutically acceptable salts.

**4.** Cyclic substituted aminomethyl compounds of the general formula IB according to claim 1, wherein $R^1$ denotes OH, O-$CH_3$ or Cl, $R^2$, $R^3$ and $R^4$ denote H, $R^5$ and $R^6$ denote $CH_3$ and X denotes $CH_2$, O or S, n is 1 or 2 and the configuration of the exocyclic double bond is E or Z, and their pharmaceutically acceptable salts.

**5.** Cyclic substituted aminomethyl compounds of the general formula IB according to one of claims 1 or 4, wherein $R^1$ denotes 3-OH, 2-O-$CH_3$, 3-O-$CH_3$ or 4-Cl, $R^2$, $R^3$ and $R^4$ denote H, $R^5$ and $R^6$ denote $CH_3$ and X denotes $CH_2$, O or S, n is 1 or 2 and the configuration of the exocyclic double bond is *E* or *Z,* and their pharmaceutically acceptable salts.

**6.** Cyclic substituted aminomethyl compounds according to one of claims 1 to 5, wherein the compounds are in the form of a mixture of the isomers with an endocyclic double bond according to the general formula IA and with an exocyclic double bond according to the general formula IB.

**7.** Cyclic substituted aminomethyl compounds of the general formulae IA and/or IB according to one of claims 1 to 6 in the form of their racemates, in the form of the pure enantiomers or in the form of mixtures of the enantiomers in any desired mixture ratio, and their pharmaceutically acceptable salts.

**8.** Cyclic substituted aminomethyl compounds according to one of claims 1 to 7, wherein the amino compounds are chosen from
[1-(4-chlorobenzyl)-3,4-dihydro-naphth-2-ylmethyl]-dimethylamine,
3-(2-dimethylaminomethyl-3,4-dihydro-naphth-1-ylmethyl)-phenol,
5-(4-chlorobenzyl)-6-dimethylaminomethyl-7,8-dihydronaphth-1-ol,
*E*-(5*RS*)-[5-(4-chlorobenzylidene)-6,7,8,9-tetrahydro-5H-benzocyclohepten-6-ylmethyl)-dimethylamine,
*Z*-(4*RS*)-[5-(4-chlorobenzylidene)-2,3,4,5-tetrahydrobenzo[b]oxepin-4-ylmethyl]-dimethylamine,
3-(4-dimethylaminomethyl-2,3-dihydro-benzo[b]oxepin-5-ylmethyl)-phenol,
*E*-(4*RS*)-3-(4-dimethylaminomethyl-3,4-dihydro-2H-benzo[b]oxepin-5-ylidenemethyl)-phenol or
Z-(4*RS*)-3-(4-dimethylaminomethyl-3,4-dihydro-2H-benzo[b]oxepin-5-ylidenemethyl)-phenol
and their pharmaceutically acceptable salts, in particular their hydrochlorides.

**9.** Process for the preparation of compounds of the general formulae IA and/or IB

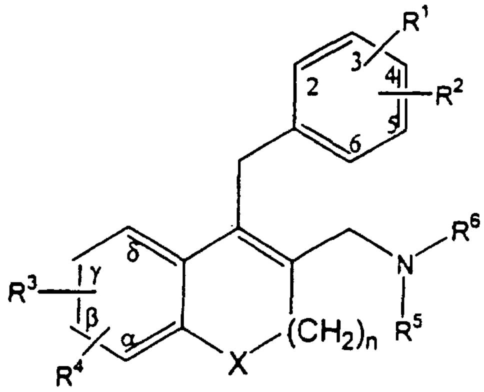

IA

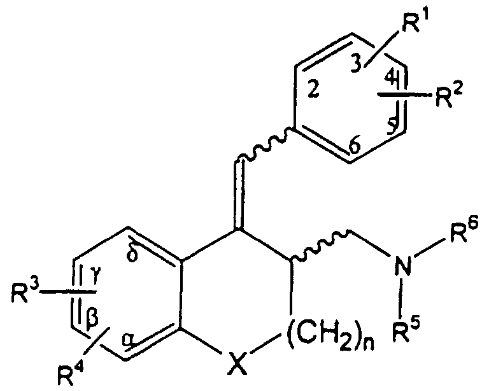

IB ,

wherein

| | |
|---|---|
| $R^1$ | denotes H, F, Cl, OH, O-$CH_3$, O-($C_{2-6}$-alkyl), O-($C_{3-7}$-cycloalkyl), $CH_3$, $C_{2-6}$-alkyl, $CH_2F$, $CHF_2$ or $CF_3$, in each case in the 2-, 3-, 4-, 5- or 6-position of the phenyl ring, |
| $R^2$ | denotes H, F, Cl, $CH_3$, $C_{2-6}$-alkyl, $CH_2F$, $CHF_2$ or $CF_3$, in each case in the 2-, 3-, 4-, 5- or 6-position of the phenyl ring, |
| $R^3$ and $R^4$ | independently of one another denote H, F, Cl, OH, O-$CH_3$, O-($C_{2-6}$-alkyl), O-($C_{3-7}$-cycloalkyl), $CH_3$, $C_{2-6}$-alkyl, $CH_2F$, $CHF_2$, $CF_3$, O-aryl, aryl or heterocyclyl, in each case in the α-, β-, γ- and/or δ-position of the aromatic ring, |
| $R^5$ and $R^6$ | independently of one another denote $CH_3$, $C_{2-6}$-alkyl, $C_{3-7}$-cycloalkyl, $CH_2$-($C_{3-7}$-cycloalkyl), aryl, ($C_{1-6}$-alkyl)-aryl, heterocyclyl or ($C_{1-6}$-alkyl)-heterocyclyl, |
| X | denotes $CH_2$, O, S, SO or $SO_2$, |
| n | is 0, 1, 2 or 3 if X denotes $CH_2$ and is 1, 2 or 3 if X denotes O, S, SO or $SO_2$ |

and the configuration of the exocyclic double bond in compounds of the general formula IB is E or Z, **characterized by** a process step (a) which comprises reaction of a tertiary alcohol of the general formula II

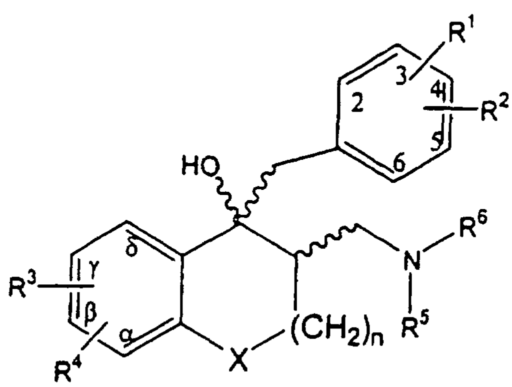

II

wherein $R^1$ to $R^6$, X and n are as defined above, with an acid.

**10.** Process according to claim 9, **characterized in that** process step (a) comprises conversion of a tertiary alcohol

of the general formula II in which at least one of the radicals $R^1$, $R^3$ and $R^4$ denotes $O-CH_3$ into compounds of the general formulae IA and/or IB in which the radicals $R^1$, $R^3$ and $R^4$ denote OH, when the corresponding radicals $R^1$, $R^3$ and $R^4$ in the tertiary alcohol of the general formula II denote $O-CH_3$, with a reagent from the group which comprises hydrogen bromide in glacial acetic acid, concentrated hydrobromic acid and methanesulfonic acid/methionine.

**11.** Process according to claim 9, **characterized in that**, before process step (a), a process step (b) is carried out which comprises conversion of a tertiary alcohol of the general formula III

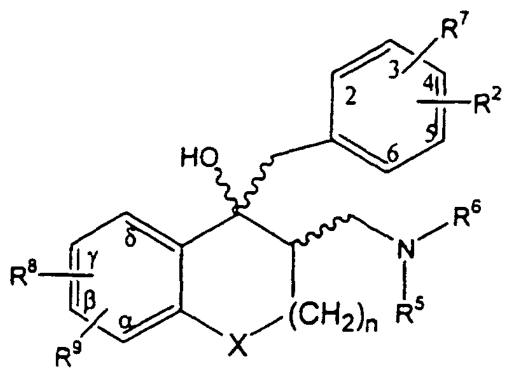

III

wherein
$R^2$, $R^5$, $R^6$, X and n are as defined in claim 9,

$R^7$ denotes H, F, Cl, $O-CH_3$, O- ($C_{2-6}$-alkyl), O- ($C_{3-7}$-cycloalkyl), $O-CH_2$-phenyl, $O-SiR^{10}R^{11}R^{12}$, wherein $R^{10}$, $R^{11}$ and $R^{12}$ independently of one another are $CH_3$, $C_{2-6}$-alkyl or phenyl, $CH_3$, $C_{2-6}$-alkyl, $CH_2F$, $CHF_2$ or $CF_3$, in each case in the 2-, 3-, 4-, 5- or 6-position of the phenyl ring,

$R^8$ and $R^9$ independently of one another denote H, F, Cl, $O-CH_3$, O-($C_{2-6}$-alkyl), O-($C_{3-7}$-cycloalkyl), $O-CH_2$-phenyl, $O-SiR^{10}R^{11}R^{12}$, wherein $R^{10}$, $R^{11}$ and $R^{12}$ independently of one another are $CH_3$, $C_{2-6}$-alkyl or phenyl, $CH_3$, $C_{2-6}$-alkyl, $CH_2F$, $CHF_2$, $CF_3$, O-aryl, aryl or heterocyclyl, in each case in the $\alpha$-, $\beta$-, $\gamma$- and/or $\delta$-position of the aromatic ring,

and at least one of the radicals $R^7$, $R^8$ and $R^9$
is $O-CH_3$, O-($C_{2-6}$-alkyl), O-($C_{3-7}$-cycloalkyl), $O-CH_2$-phenyl or $O-SiR^{10}R^{11}R^{12}$,
into a tertiary alcohol of the general formula II according to claim 9 in which $R^1$, $R^3$ and $R^4$ is in each case OH, when the corresponding radical $R^7$, $R^8$ or $R^9$ in the formula III is $O-CH_3$, O-($C_{2-6}$-alkyl), O- ($C_{3-7}$-cycloalkyl), $O-CH_2$-phenyl or $O-SiR^{10}R^{11}R^{12}$.

**12.** Process according to one of claims 9 to 11, **characterized in that**, for the preparation of the tertiary alcohols of the general formulae II or III, before process steps (a) and (b) a process step (c) is carried out, which comprises reaction of a ketone of the general formula IV

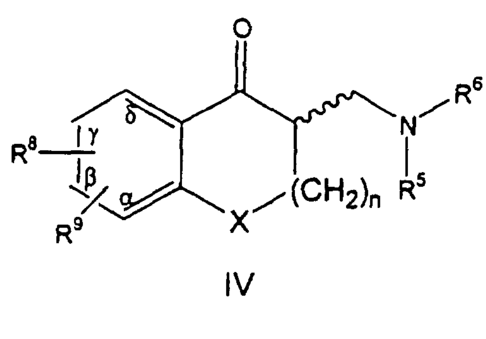

IV

wherein $R^5$, $R^6$, X and n are as defined in claim 9 and $R^8$ and $R^9$ are as defined in claim 11,

with an organometallic compound of the general formula V

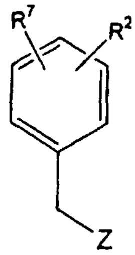

V

wherein $R^2$ is as defined in claim 9 and $R^7$ is as defined in claim 11 and Z denotes MgCl, MgBr, MgI or Li.

**13.** Process according to one of claims 9 to 12, **characterized in that** compounds of the general formulae IA and/or IB where X = S are converted in a process step (d) into compounds of the general formulae IA and/or IB where X = SO and/or $SO_2$ using an oxidizing agent.

**14.** Process according to one of claims 9 to 13, **characterized in that** after process step (a) and optionally before or after process step (d), a process step (e) is carried out, which comprises conversion of compounds of the general formula IA and/or IB in which at least one of the radicals $R^1$, $R^3$ and $R^4$ denotes O-$CH_3$ into compounds of the general formulae IA and/or IB in which the corresponding radical(s) $R^1$, $R^3$ and $R^4$ denote(s) OH.

**15.** Process according to one of claims 9 to 14, **characterized in that** after process step (a) or (d) or (e) a process step (f) is carried out, which comprises conversion of the compounds of the general formulae IA and/or IB into their pharmaceutically acceptable salts.

**16.** Process according to one of claims 9 to 15, **characterized in that** after process step (a) or before or after process step (d) or before or after process step (e) or after process step (f) a process step (g) is carried out, which comprises separation of the compounds of the general formulae IA and IB and optionally of their pharmaceutically acceptable salts.

**17.** Tertiary alcohol of the general formula II or III

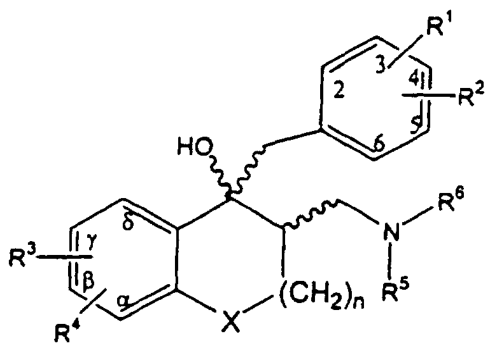

II

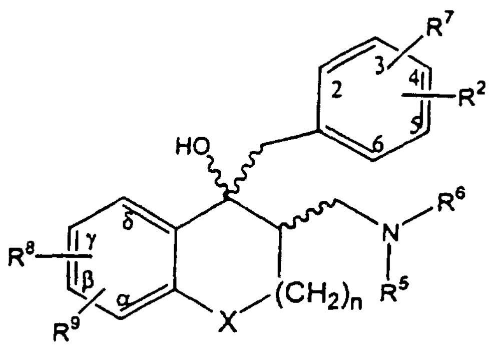

III ,

wherein $R^1$ to $R^6$, X and n are as defined in claim 9 and $R^7$ to $R^9$ are as defined in claim 11.

**18.** Medicament comprising at least one compound of the general formulae IA and/or IB or one of its pharmaceutically acceptable salts according to one of claims 1 to 8.

**19.** Use of a compound of the general formulae IA and/or IB or of one of its pharmaceutically acceptable salts according

to one of claims 1 to 8 for the preparation of a medicament for treatment of pain.

**20.** Use of a compound of the general formulae IA and/or IB or of one of its pharmaceutically acceptable salts according to one of claims 1 to 8 for the preparation of a medicament for treatment of urinary incontinence, itching, tinnitus aurium and/or diarrhoea.

**21.** Pharmaceutical composition which comprises at least one compound of the general formulae IA and/or IB or one of its pharmaceutically acceptable salts according to one of claims 1 to 8 and one or more pharmaceutical auxiliary substances.

## Revendications

**1.** Composés aminométhylés cycliques substitués de formules générales IA et/ou IB

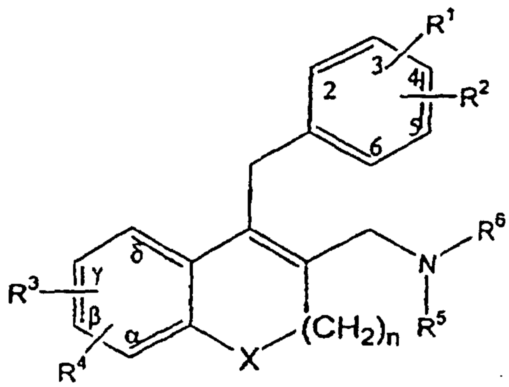

IA

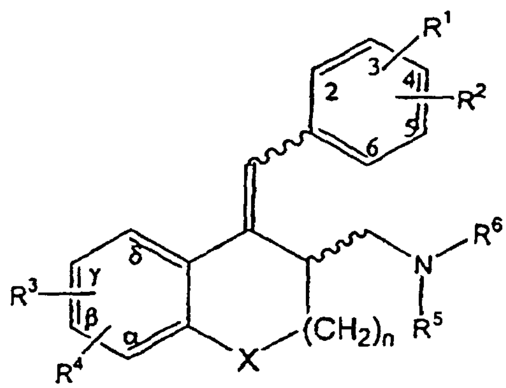

IB , 7

dans lesquelles

| | |
|---|---|
| $R^1$ | représente H, F, Cl, OH, O-$CH_3$, O-(alkyle en $C_2$ à $C_6$), O-(cycloalkyle en $C_3$ à $C_7$), $CH_3$, alkyle en $C_2$ à $C_6$, $CH_2F$, $CHF_2$ ou $CF_3$, chacun en position 2, 3, 4, 5 ou 6 du noyau phényle, |
| $R^2$ | représente H, F, Cl, $CH_3$, alkyle en $C_2$ à $C_6$, $CH_2F$, $CHF_2$ ou $CF_3$, chacun en position 2, 3, 4, 5 ou 6 du noyau phényle, |
| $R^3$ et $R^4$ | représentent indépendamment l'un de l'autre H, F, Cl, OH, O-$CH_3$, O-(alkyle en $C_2$ à $C_6$), O-(cycloalkyle en $C_3$ à $C_7$), $CH_3$, alkyle en $C_2$ à $C_6$, $CH_2F$, $CHF_2$, $CF_3$, 0-aryle, aryle ou hétérocyclyle, chacun en position α, β, γ et/ou δ du noyau aromatique, |
| $R^5$ et $R^6$ | représentent indépendamment l'un de l'autre $CH_3$, alkyle en $C_2$ à $C_6$, cycloalkyle en $C_3$ à $C_7$, $CH_2$-(cycloalkyle en $C_3$ à $C_7$), aryle, (alkyle en $C_1$ à $C_6$)-aryle, hétérocyclyle ou (alkyle en $C_1$ à $C_6$)-hétérocyclyle, |
| X | représente $CH_2$, O, S, SO ou $SO_2$, |
| n | a la valeur 0, 1, 2 ou 3, lorsque X représente $CH_2$, et la valeur 1, 2 ou 3, lorsque X représente O, S, SO ou $SO_2$, |

et la double liaison exocyclique a la configuration E ou Z dans des composés de formule générale IB,
ainsi que leurs sels acceptables du point de vue pharmaceutique.

**2.** Composés aminométhylés cycliques substitués de formule générale IA suivant la revendication 1, dans lesquels, indépendamment les uns des autres, $R^1$ représente OH, O-$CH_3$ ou Cl, $R^2$ représente H ou Cl, $R^3$ représente H ou OH, $R^4$ représente H, $R^5$ et $R^6$ représentent $CH_3$ et X représente $CH_2$, O, S ou SO, et n a la valeur 1 ou 2, ainsi que leurs sels acceptables du point de vue pharmaceutique.

**3.** Composés aminométhylés cycliques substitués de formule générale IA suivant l'une des revendications 1 ou 2, dans lesquels, indépendamment les uns des autres, $R^1$ désigne 3-OH, 2-O-$CH_3$, 3-O-$CH_3$ ou 4-Cl, $R^2$ représente H, 2-Cl ou 4-Cl, $R^3$ représente H, α-OH ou β-OH, $R^4$ représente H, $R^5$ et $R^6$ représentent $CH_3$ et X représente

$CH_2$, O, S ou SO, et n a la valeur 1 ou 2, ainsi que leurs sels acceptables du point de vue pharmaceutique.

**4.** Composés aminométhylés cycliques substitués de formule générale IB suivant la revendication 1, dans lesquels $R^1$ représente OH, O-$CH_3$ ou Cl, $R^2$, $R^3$ et $R^4$ représentent H, $R^5$ et $R^6$ représentent $CH_3$ et X représente $CH_2$, O ou S, n a la valeur 1 ou 2 et la configuration de la double liaison exocyclique est *E* ou *Z*, ainsi que leurs sels acceptables du point de vue pharmaceutique.

**5.** Composés aminométhylés cycliques substitués de formule générale IB suivant l'une des revendications 1 ou 4, dans lesquels $R^1$ désigne 3-OH, 2-0-$CH_3$, 3-O-$CH_3$ ou 4-Cl, $R^2$, $R^3$ et $R^4$ représentent H, $R^5$ et $R^6$ représentent $CH_3$ et X représente $CH_2$, 0 ou S, n a la valeur 1 ou 2 et la configuration de la double liaison exocyclique est E ou Z, ainsi que leurs sels acceptables du point de vue pharmaceutique.

**6.** Composés aminométhylés cycliques substitués suivant l'une des revendications 1 à 5, ces composés se présentant sous forme de mélange des isomères à double liaison endocyclique selon la formule générale IA et à double liaison exocyclique selon la formule générale IB.

**7.** Composés aminométhylés cycliques substitués de formules générales IA et/ou IB suivant l'une des revendications 1 à 6 sous forme de leurs racémates, sous forme des énantiomères purs ou sous forme de mélanges des énantiomères dans un rapport de mélange quelconque, ainsi que leurs sels acceptables du point de vue pharmaceutique.

**8.** Composés aminométhylés cycliques substitués suivant l'une des revendications 1 à 7, ces composés aminés étant choisis entre :

la [1-(4-chlorobenzyl)-3,4-dihydro-napht-2-ylméthyl]-diméthylamine,
le 3-(2-diméthylaminométhyl-3,4-dihydro-napht-1-yl-méthyl)-phénol,
le 5-(4-chlorobenzyl)-6-diméthylaminométhyl-7,8-dihydronapht-1-ol,
la *E*-(5*RS*)-[5-(4-chlorobenzylidène)-6,7,8,9-tétrahydro-5H-benzocycloheptène-6-ylméthyl]-diméthylamine,
la *Z*-(4*RS*)-[5-(4-chlorobenzylidène)-2,3,4,5-tétrahydrobenzo-[b]oxépine-4-ylméthyl]-diméthylamine,
le 3-(4-diméthylaminométhyl-2,3-dihydro-benzo[b]oxépine-5-ylméthyl)-phénol,
le *E*-(4*RS*)-3-(4-diméthylaminométhyl-3,4-dihydro-2H-benzo-[b]oxépine-5-ylidèneméthyl)-phénol ou
le *Z*-(4*RS*)-3-(4-diméthylaminométhyl-3,4-dihydro-2H-benzo-[b]oxépine-5-ylidèneméthyl)-phénol,

ainsi que leurs sels acceptables du point de vue pharmaceutique, en particulier leurs chlorhydrates.

**9.** Procédé de production de composés de formules générales IA et/ou IB

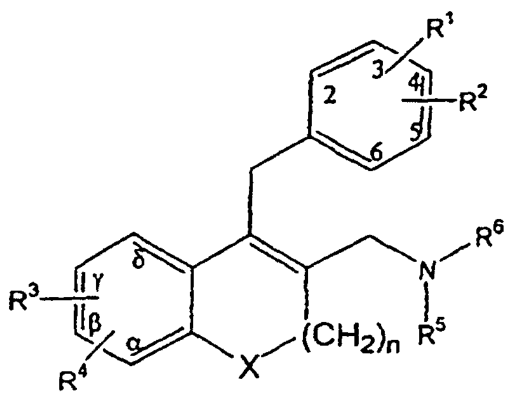

IA

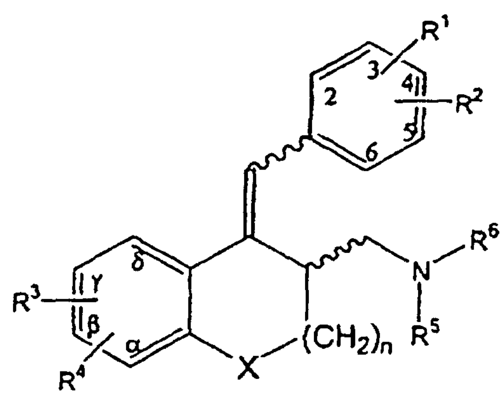

IB ,

dans lesquelles

$R^1$ représente H, F, Cl, OH, O-$CH_3$, O-(alkyle en $C_2$ à $C_6$), O-(cycloalkyle en $C_3$ à $C_7$), $CH_3$, alkyle en $C_2$ à $C_6$, $CH_2F$, $CHF_2$ ou $CF_3$, chacun en position 2, 3, 4, 5 ou 6 du noyau phényle,

R2 représente H, F, Cl, $CH_3$, alkyle en $C_2$ à $C_6$, $CH_2F$, $CHF_2$ ou $CF_3$, chacun en position 2, 3, 4, 5 ou 6 du noyau phényle,

$R^3$ et $R^4$ représentent indépendamment l'un de l'autre H, F, Cl, OH, O-$CH_3$, O-(alkyle en $C_2$ à $C_6$), O-(cycloalkyle en $C_3$ à $C_7$), $CH_3$, alkyle en $C_2$ à $C_6$, $CH_2F$, $CHF_2$, $CF_3$, O-aryle, aryle ou hétérocyclyle, chacun en position α, β, γ et/ou δ du noyau aromatique,

$R^5$ et $R^6$ représentent indépendamment l'un de l'autre $CH_3$, alkyle en $C_2$ à $C_6$, cycloalkyle en $C_3$ à $C_7$, $CH_2$-(cycloalkyle en $C_3$ à $C_7$), aryle, (alkyle en $C_1$ à $C_6$)-aryle, hétérocyclyle ou (alkyle en $C_1$ à $C_6$)-hétérocyclyle,

X représente $CH_2$, O, S, SO ou $SO_2$,

n a la valeur 0, 1, 2 ou 3, lorsque X représente $CH_2$, et la valeur 1, 2 ou 3, lorsque X représente O, S, SO ou $SO_2$,

et la double liaison exocyclique a la configuration E ou Z dans des composés de formule générale IB,
**caractérisé par** une étape (a) qui comprend la réaction avec un acide d'un alcool tertiaire de formule générale II

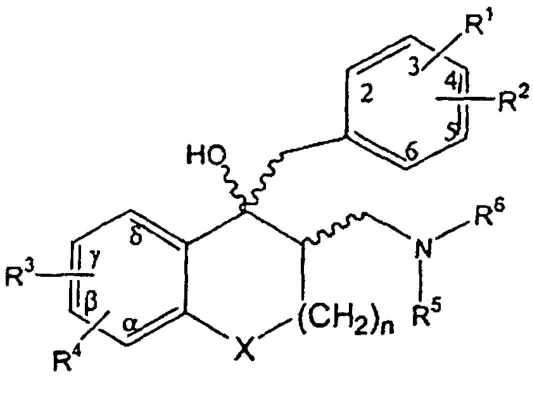

II

dans laquelle $R^1$ à $R^6$, X et n sont tels que définis ci-dessus.

**10.** Procédé suivant la revendication 9, **caractérisé en ce que** l'étape (a) comprend la réaction d'un alcool tertiaire de formule générale II, dans laquelle au moins l'un des restes $R^1$, $R^3$ et $R^4$ désigne O-$CH_3$, pour former des composés de formules générales IA et/ou IB, dans lesquelles les restes $R^1$, $R^3$ et $R^4$ désignent OH, lorsque les restes $R^1$, $R^3$ et $R^4$ correspondants dans l'alcool tertiaire de formule générale II sont des restes O-$CH_3$, avec un réactif du groupe comprenant le bromure d'hydrogène dans l'acide acétique cristallisable, l'acide bromhydrique concentré et le système acide méthanesulfonique/méthionine.

**11.** Procédé suivant la revendication 9, **caractérisé en ce que** l'étape (a) est précédée d'une étape (b) qui comprend la transformation d'un alcool tertiaire de formule générale III

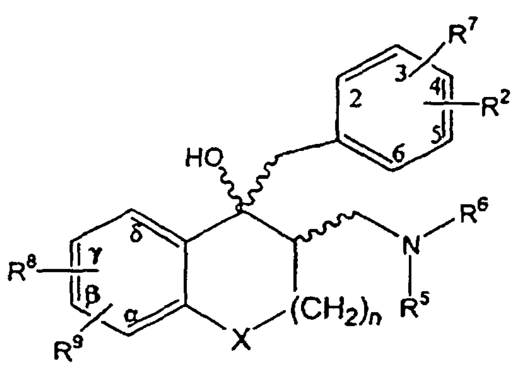

III

dans laquelle

$R^2$, $R^5$, $R^6$, X et n sont tels que définis dans la revendication 9,

| | |
|---|---|
| $R^7$ | représente H, F, Cl, O-$CH_3$, O-(alkyle en $C_2$ à $C_6$), O- (cycloalkyle en $C_3$ à $C_7$), O-$CH_2$-phényle, O-Si$R^{10}R^{11}R^{12}$, où $R^{10}$, $R^{11}$ et $R^{12}$ représentent, indépendamment les uns des autres, $CH_3$, alkyle en $C_2$ à $C_6$ ou phényle, $CH_3$, alkyle en $C_2$ à $C_6$, $CH_2F$, $CHF_2$ ou $CF_3$, chacun en position 2, 3, 4, 5 ou 6 du noyau phényle, |
| $R^8$ et $R^9$ | représentent indépendamment l'un de l'autre H, F, Cl, O-$CH_3$, O-(alkyle en $C_2$ à $C_6$), O-(cycloalkyle en $C_3$ à $C_7$), O-$CH_2$-phényle, O-Si$R^{10}R^{11}R^{12}$, où $R^{10}$, $R^{11}$ et $R^{12}$ représentent indépendamment les uns des autres $CH_3$, alkyle en $C_2$ à $C_6$ ou phényle, $CH_3$, alkyle en $C_2$ à $C_6$, $CH_2F$, $CHF_2$, $CF_3$, O-aryle, aryle ou hétérocyclyle, chacun en position $\alpha$, $\beta$, $\gamma$ et/ou $\delta$ du noyau aromatique, |
| et l'un au moins des restes $R^7$, $R^8$ et $R^9$, | représente O-$CH_3$, O-(alkyle en $C_2$ à $C_6$), O- (cycloalkyle en $C_3$ à $C_7$), O-$CH_2$-phényle ou O- Si$R^{10}R^{11}R^{12}$, |

en un alcool tertiaire de formule générale II selon la revendication 9, dans laquelle $R^1$, $R^3$ et $R^4$ représentent chacun un groupe OH, lorsque le reste $R^7$, $R^8$ ou $R^9$ correspondant dans la formule III est un reste O-$CH_3$, O-(alkyle en $C_2$ à $C_6$), O-(cycloalkyle en $C_3$ à $C_7$), O-$CH_2$-phényle ou O-Si$R^{10}R^{11}R^{12}$.

**12.** Procédé suivant l'une des revendications 9 à 11, **caractérisé en ce que**, pour la préparation des alcools tertiaires de formules générales II et III, on conduit avant les étapes (a) et respectivement (b) une étape (c) qui comprend la réaction d'une cétone de formule générale IV

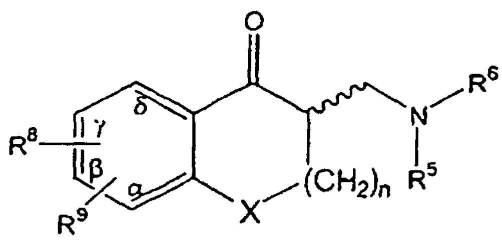

IV

dans laquelle $R^5$, $R^6$, X et n sont tels que définis dans la revendication 9, et $R^8$ et $R^9$ sont tels que définis dans la revendication 11,
avec un composé organométallique de formule générale V

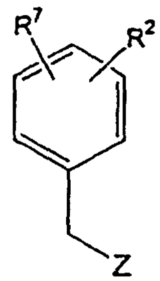

V

dans laquelle $R^2$ est tel que défini dans la revendication 9 et $R^7$ est tel que défini dans la revendication 11, et Z représente MgCl, MgBr, MgI ou Li.

**13.** Procédé suivant l'une des revendications 9 à 12, **caractérisé en ce que** des composés de formules générales IA et/ou IB, dans lesquelles X représente S, sont transformés dans une étape (d) en composés de formules générales IA et/ou IB, dans lesquelles X représente SO et/ou $SO_2$, en utilisant un agent oxydant.

**14.** Procédé suivant l'une des revendications 9 à 13, **caractérisé en ce qu'**on conduit après l'étape (a) et le cas échéant avant ou après l'étape (d) une étape (e) qui comprend la transformation de composés de formules générales IA et/ou IB, dans lesquelles l'un au moins des restes $R^1$, $R^3$ et $R^4$ désigne un reste O-$CH_3$, en composés de formules générales IA et/ou IB dans lesquelles le ou les restes $R^1$, $R^3$ et $R^4$ correspondants désignent un groupe OH.

**15.** Procédé suivant l'une des revendications 9 à 14, **caractérisé en ce qu'**on conduit après l'étape (a) ou (d) ou (e) une étape (f) qui comprend la transformation des composés de formules générales IA et/ou IB en leurs sels acceptables du point de vue pharmaceutique.

**16.** Procédé suivant l'une des revendications 9 à 15, **caractérisé en ce qu'**on conduit après l'étape (a) ou avant ou après l'étape (d) ou bien avant ou après l'étape (e) ou après l'étape (f) une étape (g) qui comprend la séparation des composés de formules générales IA et/ou IB ainsi que, le cas échéant, de leurs sels acceptables du point de vue pharmaceutique.

**17.** Alcool tertiaire de formule générale II ou III

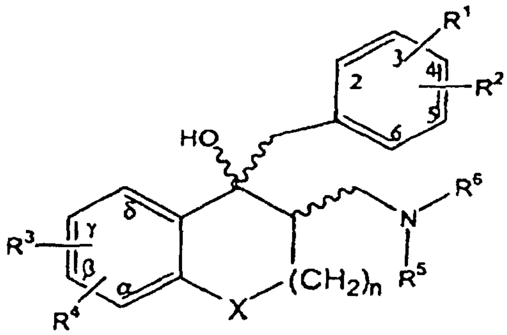

II

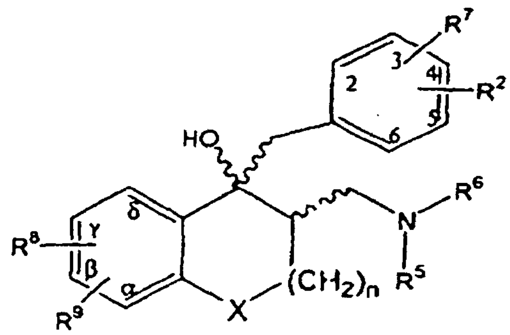

III ,

dans laquelle $R^1$ à $R^6$, X et n sont tels que définis dans la revendication 9 et $R^7$ à $R^9$ sont tels que définis dans la revendication 11.

**18.** Médicament contenant au moins un composé de formules générales IA et/ou IB ou l'un de ses sels acceptables du point de vue pharmaceutique selon l'une des revendications 1 à 8.

**19.** Utilisation d'un composé de formules générales IA et/ou IB ou d'un de ses sels acceptables du point de vue pharmaceutique selon l'une des revendications 1 à 8 pour la préparation d'un médicament destiné au traitement de la douleur.

**20.** Utilisation d'un composé de formules générales IA et/ou IB ou d'un de ses sels acceptables du point de vue pharmaceutique selon l'une des revendications 1 à 8 pour la préparation d'un médicament destiné au traitement de l'incontinence d'urine, du prurit, des acouphènes et/ou de la diarrhée.

**21.** Préparation pharmaceutique, qui contient au moins un composé de formules générales IA et/ou IB ou l'un de ses sels acceptables du point de vue pharmaceutique selon l'une des revendications 1 à 8 et une ou plusieurs substances pharmaceutiques auxiliaires.